(19) **Europäisches Patentamt / European Patent Office / Office européen des brevets**

(11) **EP 2 692 348 A1**

(12) # EUROPEAN PATENT APPLICATION
published in accordance with Art. 153(4) EPC

(43) Date of publication:
**05.02.2014 Bulletin 2014/06**

(21) Application number: **12765909.2**

(22) Date of filing: **22.03.2012**

(51) Int Cl.:
*A61K 33/00* (2006.01)       *A61P 35/00* (2006.01)
*A61P 35/02* (2006.01)       *A61P 43/00* (2006.01)

(86) International application number:
**PCT/JP2012/057360**

(87) International publication number:
**WO 2012/133114 (04.10.2012 Gazette 2012/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.03.2011 JP 2011072553**
**10.08.2011 JP 2011174852**

(71) Applicants:
• **Neochemir, Inc.**
**Kobe-shi**
**Hyogo 651-0087 (JP)**
• **CO2BE Medical Engineering K.K.**
**Hyogo 650-0047 (JP)**
• **National University Corporation Kobe University**
**Kobe-shi, Hyogo 657-8501 (JP)**

(72) Inventors:
• **AKISUE, Toshihiro**
**Kobe-shi**
**Hyogo 657-8501 (JP)**
• **MIWA, Masahiko**
**Kobe-shi**
**Hyogo 650-0047 (JP)**
• **UEHA, Takeshi**
**Kobe-shi**
**Hyogo 651-0087 (JP)**
• **TANAKA, Masaya**
**Kobe-shi**
**Hyogo 651-0087 (JP)**

(74) Representative: **Sajda, Wolf E. et al**
**Meissner, Bolte & Partner GbR**
**Postfach 86 06 24**
**81633 München (DE)**

(54) **ANTITUMOR AGENT CONTAINING CARBON DIOXIDE AS ACTIVE INGREDIENT**

(57)    An antitumor agent comprising carbon dioxide as an active ingredient of the present invention can reduce the tumor volume or eradicate the tumor of a patient who is difficult to take a surgical therapy, a chemotherapy, a radiation therapy or an immunotherapy. The antitumor agent comprising carbon dioxide as an active ingredient of the present invention is useful because the agent enables (1) the reduction in size or elimination of a tumor, (2) the suppression of tumor metastasis and also (3) the reduction in volume or elimination of a tumor at a part different from the part where carbon dioxide is transdermally absorbed by the transdermal absorption of carbon dioxide through the skin near the tumor or the skin far from the tumor. When the antitumor agent comprising carbon dioxide as an active ingredient of the present invention is used in combination with a surgical therapy, a chemotherapy, a radiation therapy or an immunotherapy, the effect can be potentiated or side effects can be reduced as compared with a monotherapy or a multidisciplinary therapy thereof

EP 2 692 348 A1

**Description**

TECHNICAL FIELD

The present invention is related to an antitumor agent comprising carbon dioxide as an active ingredient.

BACKGROUND ART

Patent Document 1 describes that carbon dioxide is effective for treating disorders etc. such as (1) to (10) as follows:

(1) itching accompanying mucocutaneous diseases or mucocutaneous disorders such as athlete's foot, insect bite, atopic dermatitis, nummular eczema, xeroderma, seborrheic eczema, urticaria, prurigo, housewives' eczema, acne vulgaris, impetigo, folliculitis, carbuncle, furunculosis, phlegmon, pyoderma, psoriasis, ichthyosis, palmoplantar keratoderma, lichen, pityriasis, wound, burn, rhagades, erosion and chilblain; mucocutaneous injuries such as decubitus ulcer, wound, bum, angular stomatitis, stomatitis, skin ulcer, rhagades, erosion, chilblain and gangrene;
(2) incomplete takes of skin graft, skin flap, etc.;
(3) dental diseases such as gingivitis, alveolar pyorrhea, denture ulcer, nigricans gingiva, and stomatitis;
(4) skin ulcers, cryesthesia and numbness caused by peripheral circulatory disorders such as thromboangitis obliterans, arteriolosclerosis obliterans, diabetic peripheral circulatory disorder, and varicosis in lower extremity;
(5) musculoskeletal diseases such as chronic rheumatoid arthritis, cervico-omo-brachial syndrome, myalgia, arthralgia and lumbago;
(6) nervous system diseases such as neuralgia, polyarthritis and subacute myelo-optic neuropathy;
(7) keratoses such as psoriasis, corns, callouses, ichthyosis, palmoplantar keratoderma, lichen, and pityriasis;
(8) suppurative skin diseases such as acne vulgaris, impetigo, folliculitis, carbuncles, furuncules, phlegmon, pyoderma and suppurative eczema ;
(9) constipation caused by loss of reflection of defecation; and
(10) suppression of hair regrowth after depilation (treatment of unwanted hair); cosmetic  troubles with the skin or hair such as freckles, rough skin, muddy complexion, faded skin complexion and loss of hair glossiness, etc.

In order to achieve the amelioration of the above mentioned disorder, means for carbon dioxide-absorbing such as a composition for preparing carbon dioxide for external use (see Patent Document 2 and 3), a carbon dioxide composition for external use (see Patent Document 4), a compositon for preparing a carbon dioxide gel for external use (see Patent Document 5), a material for preparing a carbon dioxide agent for external use (see Patent Document 6), and a carbon dioxide external administration device (see Patent Document 7 and 8) are disclosed.
Also use of the mean for absorbing carbon dioxide for strengthening muscle is disclosed (Patent Document 9).
However, an antitumor agent comprising carbon dioxide as an active ingredient is never known.
In general there are three major methods of treating tumors, which are surgery therapy, chemotherapy and radiation therapy. Also the modern policy of treating tumors is multidisciplinary therapy, in which multiple methods for treating tumor are combined. Further immunotherapy is now attracting attention as the fourth method for treating tumors.
The surgery therapy has developed from long time ago as a remedy for treating tumors and is a core of the multidisciplinary therapy at present. On the contrary, rapid proliferation of tumor cells and/or metastasis are sometimes observed after surgical operation, and maintaining a quality of life (QOL) is often hindered by loss of vital function accompanied with resection of the tumor or dysfunction after surgery. Also physical strength for the surgery is required for a patient receiving this therapy, and it is difficult to apply the therapy to an aged person or a patient who decreased physical strength through a long treatment.
In chemotherapy, chemical substance (an anticancer agent) is administrated to suppress tumor cell division and kill the tumor cell. After injection or oral administration, the anticancer agent is delivered from head to foot through blood vessels, and attacks tumor cells. It is possible to attack antitumor cells throughout the body and therefore effective in systemic treatment.
However, an anticancer agent does not only attack tumor cells but has a side effect to damage normal cells, and there is no selective toxicity not to affect normal cells in the anticancer agent available at present. In order to improve the selective toxicity, a lot of studies are now in progress but to date we do not have an anticancer agent without a side effect or no means to prevent the side effect completely.
In radiotherapy, X ray or gamma ray etc. is radiated to suppress tumor cell division and control the proliferation of tumor cells. But the radiation gives damage to not only tumor cells but also normal cells and radiation method is continuously studied so that it gives maximal effect only to the tumor cells. Indeed, radiotherapy is not free from side effects and some side effects are observed just after treatment (acute stage), and other side  effects come out years later(late stage). In general, typical examples of the side effects include inflammation of the skin or mucosa, bone-marrow disorder, fatigue, anorexia and the like and much or less a side effect is observed in most cases.

In immunotherapy, immune system of a patient is activated and tumor is treated by making immune cells or proteins attack tumor cells. Usually lentinan, krestin (trade mark), picibanil (trade mark), sizofiran, BCG or tumor vaccine etc. are administrated to activate the immune system. Also methods such as destroying tumor cells by activated patient's lymphocytes that are cultivated outside of the body, or attacking tumor cells directly by anti-tumor antibody are studied.

PRIOR ART REFERENCES

PATENT DOCUMENTS

| Patent Document 1: | JP 2000-319 187 A |
| Patent Document 2 | WO 2002/80941 A |
| Patent Document 3 | WO 2006/80398 A |
| Patent Document 4 | WO 2003/57228 A |
| Patent Document 5 | WO 2005/16290 A |
| Patent Document 6 | WO 2004/04745 A |
| Patent Document 7 | WO 2004/02393 A |
| Patent Document 8 | WO 2007/112726 A |
| Patent Document 9 | JP 2009-120 606 A |

DISCLOSURE OF THE INVENTION

The objective of the present invention is to provide with an antitumor agent comprising carbon dioxide, which is effective in a simple and safe manner. Another objective is to provide with an antitumor agent comprising carbon dioxide administrated in the combination with other therapies such as surgical therapy, chemotherapy, radiotherapy or immunotherapy etc., the effect of which is potentiated and/or the side effects of which is suppressed.

MEANS TO SOLVE THE PROBLEM

Inventors of the present invention have found that tumor is reduced or destroyed and tumor metastasis is suppressed by absorption of carbon dioxide into the body, and completed the present invention.

The present invention is an antitumor agent by which (1) tumor is reduced or disappeared and (2) tumor metastasis is suppressed by absorption of carbon dioxide.

In the present invention, both of a malignant tumor and a benign tumor are included in the group of tumors.

Examples of the malignant tumor include malignant tumors of brain nerves such as glioma and meningioma; malignant tumors of oral cavity, nose, nasal cavity, larynx or pharynx such as tongue cancer, gingival cancer, malignant lymphoma, malignant melanoma, maxillary cancer, nose cancer, nasal cavity cancer, laryngeal cancer and pharyngeal cancer; malignant tumors of thyroid such as thyroid papillary carcinoma, follicular thyroid cancer and medullary thyroid carcinoma; malignant tumors of respiratory tract such as squamous cell carcinoma, adenocarcinoma, alveolar cell carcinoma, large cell anaplastic carcinoma, small cell anaplastic carcinoma and carcinoid; malignant tumors of breast such as breast cancer, mammary Paget's disease and breast sarcoma; malignant tumors of blood such as acute myeloid leukemia, acute promyelocytic leukemia, acute myelomonocytic leukemia, acute monocytic leukemia, acute lymphocytic leukemia, acute undifferentiated leukemia, chronic myeloid leukemia, chronic lymphocytic leukemia, adult T-cell leukemia, malignant lymphoma, multiple myeloma and primary macroglobulinemia; malignant tumors of digestive organ such as esophageal cancer, stomach cancer, stomach-large intestine leiomyosarcoma, stomach-intestine malignant lymphoma, pancreatic-gallbladder cancer, duodenal cancer, colon cancer, primary liver cancer and hepatoblastoma; malignant tumors of female genital such as uterine epithelium cancer, uterine cervix squamous epithelium cancer, uterine adenocarcinoma, uterine gland squamous epithelium cancer, uterine body adenoacanthoma, uterine sarcoma, uterine cancer sarcoma, invasive hydatidiform mole, malignant chorioepithelioma of uterus, malignant melanoma of uterus, ovarian cancer and mesodermal mixed tumor; malignant tumors of urinarye organ such as kidney cancer, transitional cell cancer of renal pelvis, ureter transitional cell carcinoma, bladder papillary carcinoma, bladder transitional cell carcinoma, prostate cancer, urinary tract squamous cell carcinoma, urethral adenocarcinoma and Wilms'tumor; malignant tumors of musculoskeletal such as rhabdomyosarcoma, fibrosarcoma, osteosarcoma, chondrosarcoma, synovial sarcoma, mucus sarcoma, liposarcoma, Ewing sarcoma and multiple myeloma; malignant tumors of skin such as skin squamous cell carcinoma, basal cell skin cancer, skin Bowen's disease, Paget's disease of skin and cutaneous malignant melanoma; and malignant tumors of body cavity such as malignant mesothelioma cancer, malignant melanoma, metastatic adenocarcinoma, metastatic squamous cell carcinoma, metastatic sarcoma, leukemia, malignant lymphoma and neuroblastoma; or benign tumors of brain such as meningioma, pituitary adenoma and nerve sheath tumor; benign tumors of subcutaneous tissue such as nevus, suspended fibroma, hemangioma, vascular birthmark, lymphangioma, pyogenic granuloma, seborrheic keratosis, dermatofibroma, keratoacanthoma, keloid, lipoma, brown lipoma, neurofibromatosis, schwannoma and atheroma; benign tumors of bone such as osteocartilaginous exostosis, chondroma, chondroblastoma, osteoid osteoma and giant cell tumor; benign tumors of intestinal such as lipoma, fibroids and polyp; benign tumors of liver such as hepatocellular adenoma and bile duct adenoma; benign tumors of bile duct such as papilloma and villous adenoma; benign tumors of ear such as ear mushroom and cholesteatoma; and benign tumors of oral cavity or salivary gland such as benign pleomorphic adenoma, monomorphic adenoma, oncocytoma, papillary cystadenoma lymphoma and ameloblastoma.

The present invention is an antitumor agent comprising carbon dioxide as an active ingredient.

The present invention is an antitumor agent comprising carbon dioxide as an active ingredient, in which carbon dioxide is locally administrated.

The present invention is an antitumor agent comprising carbon dioxide as an active ingredient, in which carbon dioxide is transdermally administrated through the skin near the tumor.

The present invention is an antitumor agent comprising carbon dioxide as an active ingredient, in which carbon dioxide is transdermally administrated through the skin far from the tumor.

The present invention is an antitumor agent comprising carbon dioxide as an active ingredient, which suppresses the metastasis of a tumor.

The present invention is an antitumor agent comprising carbon dioxide as an active ingredient, which is used in combination with one or more of surgical therapy, chemotherapy, radiotherapy and immunotherapy of a tumor in order to enhance the antitumor effect and/or suppress the side effects.

The following embodiments are preferable in this invention.

The present invention is an antitumor agent comprising carbon dioxide as an active ingredient, wherein a means of absorbing carbon dioxide is a carbon dioxide external administration device.

The preferable example of the present invention is an antitumor agent comprising carbon dioxide as an active ingredient, wherein a means of absorbing carbon dioxide is a carbon dioxide external administration device, including a sealing enclosure member capable of sealing off a body surface from outside air; a supply unit for supplying carbon dioxide to inside of the sealing enclosure member and a medium to dissolve carbon dioxide inside of the sealing enclosure member above, and being equipped with an absorption aid that assists transdermal absorption of carbon dioxide.

The preferable example of the present invention is an antitumor agent comprising carbon dioxide as an active ingredient, wherein a means of absorbing carbon dioxide is a carbon dioxide external administration device, including a sealing enclosure member capable of sealing off a body surface from outside air; a supply unit for supplying carbon dioxide into the inside of the sealing enclosure member, and perspiration promoting means for promoting perspiration at the body surface inside the sealing enclosure member.

The preferable example of the present invention is an antitumor agent comprising carbon dioxide as an active ingredient, wherein a means of absorbing carbon dioxide is a carbon dioxide external preparation.

The preferable example of the present invention is an antitumor agent comprising carbon dioxide as an active ingredient, wherein the carbon dioxide external preparation is one or more selected from a group consisting of:

(1) a carbon dioxide external agent including a medium which dissolves carbon dioxide and carbon dioxide is dissolved therein;
(2) a carbon dioxide external agent consisting of a solid material containing an acid, and a liquid material containing a carbonate salt, the preparation being obtained by mixing the solid material above and the liquid material above at use;
(3) a carbon dioxide external agent consisting of a solid material containing a carbonate salt, and a liquid material containing an acid, the preparation being obtained by mixing the solid material above and the liquid material above at use;
(4) a carbon dioxide external agent obtained by adding water to a solid material containing an acid and a carbonate salt at use;
(5) a carbon dioxide external agent consisting of a liquid material containing an acid, and a liquid material containing a carbonate salt, which is obtained by mixing these liquid materials at use;
(6) a carbon dioxide external agent obtained from a composition for preparing an external carbon dioxide agent, comprising a granular material containing a water-soluble acid, a thickener and a water-soluble dispersant as the essential components wherein said thickener is mixed with the water-soluble acid and the water-soluble dispersant; and a viscous material containing a carbonate, water and a thickener as the essential components, which is to be mixed with the granular material at use;

(7) a carbon dioxide external agent obtained from a composition for preparing carbon dioxide gel for external use characterized by comprising granular material (A) and viscous material (B) to be mixed with the granular material (A):

(A) a granular material comprising a weak acid and a calcium ion trapping agent as essential components;
(B) a viscous material comprising calcium carbonate, a gelling agent to be gelated with calcium ions and water as essential components,

(8) a carbon dioxide external agent obtained from a composition for preparing a carbon dioxide external agent characterized by comprising a substance generating an acid after being hydrolyzed, a carbonate, a thickener, and water as essential components,
(9) a composition of carbon dioxide for external use (synonymous with carbon dioxide external agent) characterized by a viscous material comprising water and a thickener at least, in which carbon dioxide is dissolved substantially in a non-bubble state; or a composition of carbon dioxide for external use (synonymous with carbon dioxide external agent) comprising a fermenting micro-organism and metabolite thereof, a thickener, water and carbon dioxide at least, and
(10) a carbon dioxide external agent obtained from a material for formation of carbon dioxide external preparation characterized by comprising a base agent that comprises a polymeric three-dimensional network structure impregnated with a viscous material containing at least an acid and water, and is made to contact with skin at use, and a reactant that contains at least a carbonate, and is made to contact with the base agent at use so as to generate carbon dioxide.

The preferable example of the present invention is an antitumor agent comprising carbon dioxide as an active ingredient, wherein the means to supply carbon dioxide is local administration of a mixture of vapor and/or suspended droplet of medium in which carbon dioxide dissolves and carbon dioxide.

The present invention is an antitumor agent comprising carbon dioxide as an active ingredient, wherein reduction or disappearance of a tumor and control of metastasis are achieved simultaneously.

EFFECT OF THE INVENTION

An antitumor agent comprising carbon dioxide as an active ingredient of the present invention can reduce in size or eliminate the tumor of a tumor patient who has difficulty in undergoing a surgical therapy, a chemotherapy, a radiation therapy or an immunotherapy almost without causing side effects.

In addition, the antitumor agent comprising carbon dioxide as an active ingredient of the present invention is useful because the agent enables (1) the reduction in size or elimination of a tumor, and (2) the suppression of tumor metastasis, by not only the local absorption of carbon dioxide through the skin near the tumor, but also through the skin far from the tumor. When the antitumor agent comprising carbon dioxide as an active ingredient of the present invention is used in combination with a surgical therapy, a chemotherapy, a radiation therapy or an immunotherapy, the effect can be potentiated or side effects can be reduced as compared with a monotherapy or a multidisciplinary therapy thereof

In particular, when the antitumor agent comprising carbon dioxide as an active ingredient of the present invention is used in combination with a surgical therapy, more improved therapeutic outcome than the surgical therapy alone can be achieved on the suppression of rapid proliferation of tumor cells and tumor metastasis, or on the reduction in size of a tumor.

When the antitumor agent comprising carbon dioxide as an active ingredient of the present invention is used in combination with a chemotherapy, the same effect as those achieved by the chemotherapeutic agent alone can be obtained at less dose of the chemotherapeutic agent compared to administration of the chemotherapeutic agent alone, and therefore, less side effects are obtained. And when the same dose of the chemotherapeutic agent as those used in a monotherapy is administered, stronger effect can be obtained.

When the antitumor agent comprising carbon dioxide as an active ingredient of the present invention is used in combination with a radiation therapy, the same effect as those achieved by the radiation alone can be obtained at less radiation dose compared to the radiation alone, and therefore, less side effects are obtained. And when the same dose of  the radiation as those used in a monotherapy is irradiated, stronger effect can be obtained.

When the antitumor agent comprising carbon dioxide as an active ingredient of the present invention is used in combination with an immunotherapy, the same effect as those achieved by the immunotherapeutic agent alone can be obtained at less dose of the immunotherapeutic agent compared to the administration of the immunotherapeutic agent alone, and therefore, less side effects are obtained. And when the same dose of the immunotherapeutic agent as those used in a monotherapy is administered, stronger effect can be obtained.

Tumor therapy can be easily done even in a medical institution that has no hospitalization facility by using the antitumor agent comprising carbon dioxide as an active ingredient of the present invention because the agent enables carbon dioxide to be absorbed into the targeted part when it is administered by using a portable means for topical absorption of carbon dioxide such as a composition for preparing an external carbon dioxide agent or a carbon dioxide external administration device. A therapy with the antitumor agent comprising carbon dioxide as an active ingredient of the present invention requires less cost than those required by a surgical therapy, a chemotherapy, a radiation therapy or an immunotherapy because the active ingredient, that is, carbon oxide is inexpensive.

Of course, the antitumor agent comprising carbon dioxide as an active ingredient of the present invention can also be applied to animals.

BRIEF DESCRIPTION OF DRAWINGS

Figure 1 Antitumor effects by twice-weekly (a total of 4 times) administration of the antitumor agent comprising carbon dioxide as an active ingredient to the implanted tumor in nude mice are shown.

Figure 2 Antitumor effects by twice-weekly administration (a total of 3 times) of the antitumor agent comprising carbon dioxide as an active ingredient to the implanted tumor in nude mice are shown.

Figure 3 Antitumor effects by twice-weekly administration of the antitumor agent comprising carbon dioxide as an active ingredient to the implanted tumor in nude mice are shown.

Figure 4 Antitumor effects by 5 consecutive days administration of the antitumor agent comprising carbon dioxide as an active ingredient with to the implanted tumor in nude mice from the 1st day of the treatment are shown.

Figure 5 Combined effects of administration of the antitumor agent comprising carbon dioxide as an active ingredient and a chemotherapeutic agent to the implanted tumor in nude mice are shown.

Figure 6 A schematic drawing of the constitution of a carbon dioxide external administration device used in the present invention is shown.

Figure 7 A schematic drawing of the constitution of a carbon dioxide external administration device used in the present invention is shown.

Figure 8 A schematic drawing of the constitution of a carbon dioxide external administration device used in the present invention is shown.

Figure 9 A schematic drawing of the constitution of a carbon dioxide external administration device used in the present invention is shown.

Figure 10 A schematic drawing of the constitution of a carbon dioxide external administration device used in the present invention is shown.

Figure 11 Combined effects of administration of the antitumor agent comprising carbon dioxide as an active ingredient and a chemotherapeutic agent to the implanted tumor in nude mice are shown.

Figure 12 Side-effects (weight change) of combined administration of the antitumor agent comprising carbon dioxide as an active ingredient and a chemotherapeutic agent to the implanted tumor in nude mice are shown.

Figure 13 Combined effects of administration of the antitumor agent comprising carbon dioxide as an active ingredient and radiation to the implanted tumor in nude mice are shown.

Figure 14 Antitumor effects of a single dose of the antitumor agent comprising carbon dioxide as an active ingredient or a chemotherapeutic agent; as well as antitumor effect of combined administration of a single dose of both agents to the implanted tumor in nude mice are shown.

Figure 15 Side-effects (weight change) by a single dose of the antitumor agent comprising carbon dioxide as an active ingredient or a chemotherapeutic agent; as well as side-effects (weight change) of combined administration of a single dose of both agents to the implanted tumor in nude mice are shown.

Figure 16 Metastasis suppression effects (lung weight) of administration of the antitumor agent comprising carbon dioxide as an active ingredient to the implanted highly lung-metastatic tumor in nude mice are shown.

Figure 17 Metastasis suppression effects (lung metastasis rate) of administration of the antitumor agent comprising carbon dioxide as an active ingredient to the implanted highly lung-metastatic tumor in nude mice are shown.

Figure 18 Antitumor effects of administration of the antitumor agent comprising carbon dioxide as an active ingredient to the implanted highly lung-metastatic tumor in nude mice are shown.

DISCLOSURE OF THE INVENTION

There is no limitation in the antitumor agent of the present invention comprising carbon dioxide as an active ingredient, as long as carbon dioxide is absorbed into the body. Examples of the means for absorption of carbon dioxide into the body include a carbon dioxide external administration device, carbon dioxide compositions for external use, carbon dioxide spring, a mixture of vapor and/or suspended droplet of a medium in which carbon dioxide dissolves and the like, and one or more of these means can be used.

The carbon dioxide external administration device used in the present invention is a means for transdermally absorbing gaseous carbon dioxide, in which a part of the body absorbing carbon dioxide is enclosed with a sealing enclosure member capable of sealing off a body surface from outside air, and an absorption route of carbon dioxide is built by applying an absorption aid containing a medium which dissolves carbon dioxide to the skin; or the device is a means for transdermally absorbing carbon dioxide in which perspiration on the body surface is enhanced inside the sealing enclosure membrane by a heater or a heat-generating substance, the perspiration is used as an absorption aid of carbon dioxide while carbon dioxide is supplied into the inside of the sealing enclosure membrane by a mixture of vapor and/or suspended droplet of a medium which dissolves carbon dioxide. Needless to say, the carbon dioxide external administration device used in the present invention is not limited to these means, but includes all means for absorption of carbon dioxide transdermally.

The carbon dioxide composition for external use in the present invention means a liquid formulation or a semisolid formulation applicable to the skin, containing a medium which dissolves carbon dioxide and carbon dioxide is dissolved therein; or a formulation of an adhesive skin patch impregnated with the above mentioned liquid or the semisolid. The carbon dioxide composition for external use may be directly applied to the skin or a patch such as a non-woven cloth impregnated with the composition may be applied to. Or a necessary part of the body may be soaked in the large amount of the carbon dioxide composition for external use.

The mixture of vapor and/or suspended droplet of a medium which dissolves carbon dioxide and carbon dioxide in the present invention means a mixture of a vapor of a medium which dissolves carbon dioxide and all or a part of which vaporized at room temperature and/or suspended droplet of said medium and carbon dioxide. The suspended droplet is microscopic droplet of a liquid floating in the air and it is called "fog" when the liquid is water.

Examples of the carbon dioxide external administration device, which are one of means for absorption of carbon dioxide into the body in the present invention, are shown below:

(1) a carbon dioxide external administration device, comprising a sealing enclosure member capable of sealing a body surface from ambient air, supply unit for supplying carbon dioxide into the inside of the sealing enclosure member, and an absorption aid capable of assisting the transdermal or transmucosal absorption of carbon dioxide inside the sealing enclosure member (WO 2004/02393), and

(2) a carbon dioxide external administration device characterized by including: a sealing enclosure member capable of sealing the body surface of a human or an animal from the outside air; a supply unit for supplying carbon dioxide into the inside of the sealing enclosure member; and perspiration promoting means for promoting perspiration at the body surface inside the sealing enclosure member (WO 2008/047829 A).

Embodiments of the present invention when the two carbon dioxide external administration devices (1) and (2) described above are explained below with reference to the drawings. In addition, the same sign means the same element or corresponding element in each drawing.

The carbon dioxide external administration device (1):

The carbon dioxide external administration device 1 comprises at least a sealing enclosure member 2 capable of sealing a body surface from the outside air, a supply means 3 for supplying carbon dioxide into the inside of the sealing enclosure member 2, and an absorption aid 4 that assists transdermal or transmucosal absorption of the carbon dioxide inside the sealing enclosure member 2. Note, however, that the sealing enclosure member 2 does not necessarily have to seal the body surface from the outside air completely, but rather it is sufficient to so long as the carbon dioxide concentration inside the sealing enclosure member 2 can be maintained above a certain level by continuously supplying a small amount of carbon dioxide into the sealing enclosure member 2. In addition, the absorption aid 4 is depicted as a sheet in the drawing, but any liquid or semisolid may be applied to the skin on the condition that carbon dioxide can be dissolved and it is applicable to the skin. Examples of a formulation of the liquid or semisolid include liquid, cream, paste or gel and one or more than two of the formulations may be applied to.

Also there are no particular limitations on the shape, materials and so on of the sealing enclosure member 2, so long as the sealing enclosure member 2 covers the skin or mucosa and has a space therein, in which carbon dioxide is held.

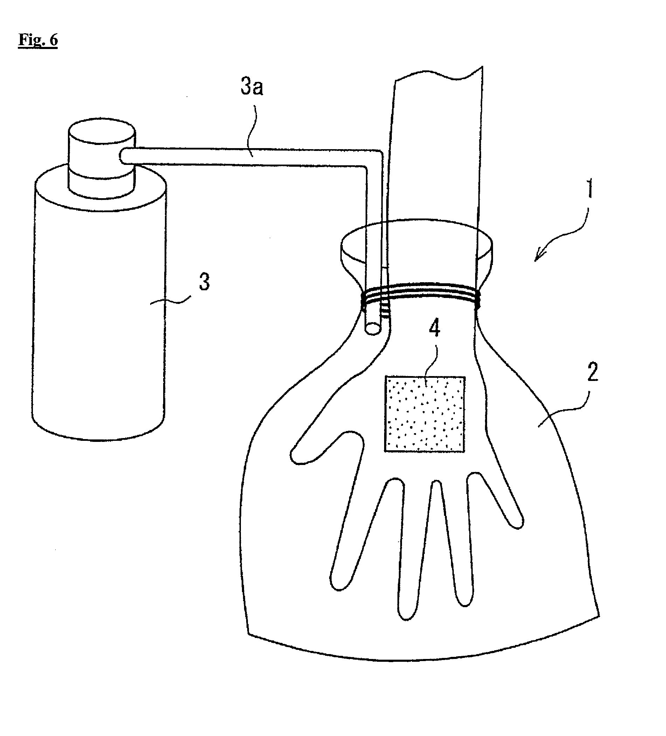

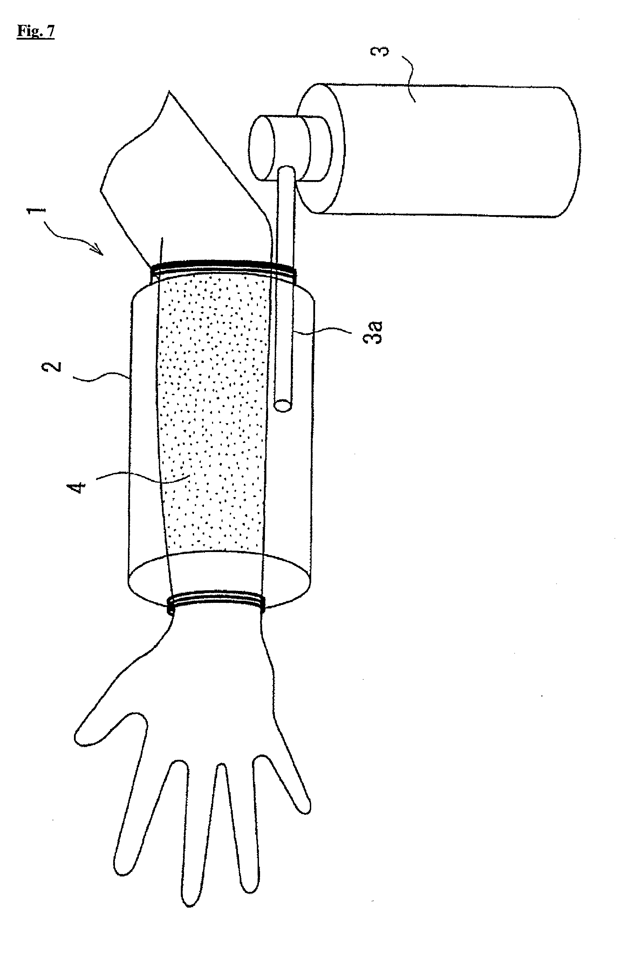

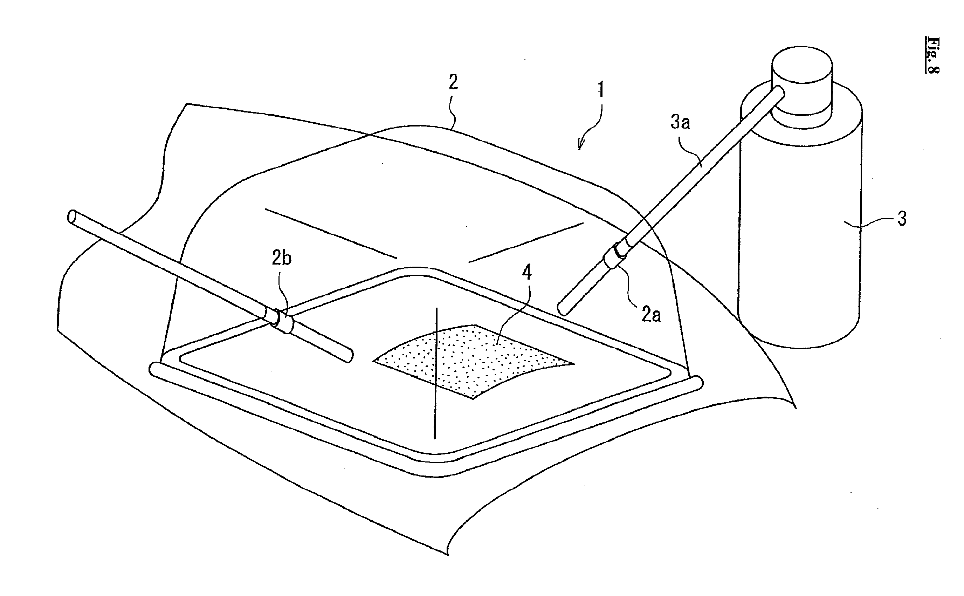

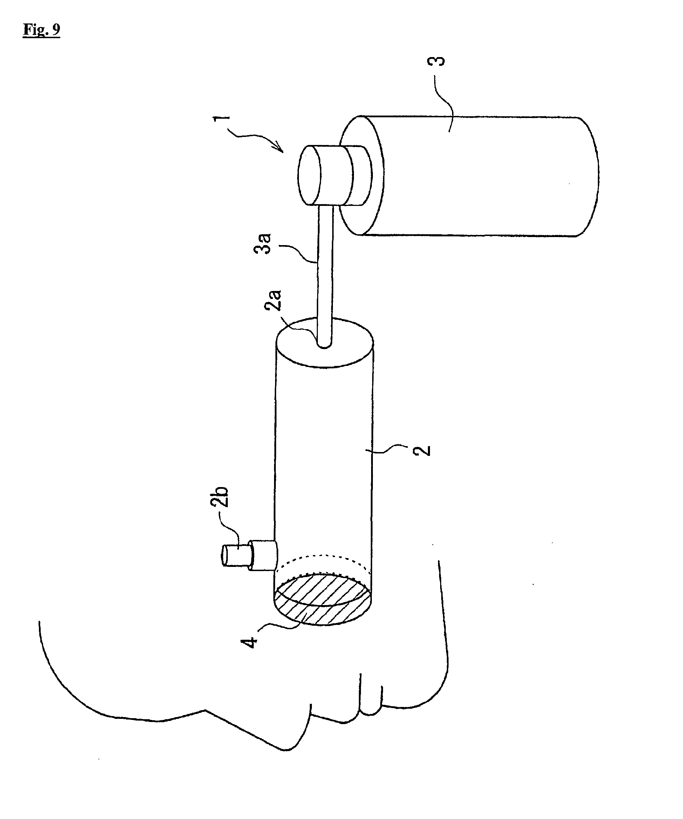

Regarding the shape, for example, a bag that can completely pack a hand (or a foot) as shown in Fig. 6, and a tubular bag that wraps around an arm (or a leg) as shown in Fig. 7 can be used. Moreover, as the shape of the sealing enclosure member 2, a vessel having an open hemline that adheres to a relatively large area of the skin surface such as the abdomen or the like as shown in Fig. 8, or a cup-shaped tubular body that has one end thereof open and the other end thereof made to be a part connecting to the supply means 3 as shown in Fig. 9 can be used. Of course, the shape of the sealing enclosure member 2 is not limited to these examples.

There are no particular limitations on the materials of the sealing enclosure member 2 so long as these materials are impermeable to gases; materials can be selected from metal, plastic, rubber, glass and so on and used as appropriate in accordance with the purpose, the part of application and so on.

In the case that the sealing enclosure member 2 is one made of only a non-elastic and hard material such as metal or glass (hereinafter referred to as "rigid sealing enclosure member"), a shape having therein a certain sealed space surrounding the part of application is preferable. The part contacting with the skin or mucosa preferably has a shape conforming to the part of application so that carbon dioxide will not leak out; combining  with an elastic material such as rubber or a resin for the part contacting with the skin or mucosa is preferable since it will be possible to flexibly respond to the part of application The combination with a material that has higher flexibility and excellent adhesion to the skin or mucosa such as a viscoelastic gel is more preferable.

Moreover, to fill carbon dioxide into the sealed space in which the skin or mucosa is sealed by the rigid sealing enclosure member, the enclosure member preferably has a gas outlet therein. When injecting carbon dioxide into the sealed space, air in the sealed space escapes out from a gap in the part contacting with the skin or mucosa and the air is replaced with carbon dioxide. But the adjustment of the size of the gap is so hard that it is difficult to carry out the replacement reliably, and the amount of carbon dioxide required for the replacement is not fixed. For example, by providing a gas outlet in, a position furthest from the gas inlet, the replacement of the air with carbon dioxide can be carried out efficiently in a short period of time.

The gas inlet 2a in the rigid sealing enclosure member (see Figs 8 and 9) has connected thereto a tube 3a that is connected to the supply means 3; there are no particular limitations on the gas inlet 2a so long as gas will not leak therefrom, but one equipped with a check valve for preventing back flow of gas is more preferable. Regarding the gas outlet 2b in the rigid sealing enclosure member (see Figs. 8 and 9), a gap in the part contacting with the skin or mucosa may be used, but to carry out the replacement of the air inside the enclosure member with carbon dioxide reliably and efficiently, a gas outlet equipped with a check valve for preventing back flow of gas is more preferable.

In the case that the sealing enclosure member 2 is one made of a material that is a flexible material such as a rubber, a resin or a soft plastic, especially, a material that has a shape holding ability, by which the shape is barely kept by some external force (hereinafter referred to as a "flexible sealing enclosure member"), the part contacting with the skin or mucosa is also flexible, and hence the above mentioned material can be used as is. However, the combination with a material that has yet higher flexibility and excellent adhesion to the skin or mucosa such as a viscoelastic gel for the part contacting with the skin or mucosa is more preferable. Moreover, with such a flexible sealing enclosure member, the same gas inlet and gas outlet as those in the case of a rigid sealing enclosure member can be used.

In the case that the sealing enclosure member 2 is one made of a material that is a flexible material such as a rubber or a resin, especially a material that is elastic highly like a balloon (hereinafter referred to as an 'elastic sealing enclosure member'), this can be used in the form of a tube, a bag or the like, with the skin or mucosa being sealed by binding the mouth of the tube, bag or the like covering the desired part, or providing a retractable opening/closing mouth in which rubber, a spring or the like is placed in the mouth.

Moreover, in the case of a tubular elastic sealing enclosure member for which the part of application is made to be, for example, an arm, the circumference of the tube may be  made to be approximately the same as that of the arm, or may be made smaller, with the skin or mucosa being sealed in a state with the sealing enclosure member adhering to the skin or mucosa, or a state with only a very small space between the sealing enclosure member and the skin or mucosa; carbon dioxide may then be injected between the sealing enclosure member and the skin or mucosa so as to expand the sealing enclosure member, and the certain space in which carbon dioxide may be held is created by the carbon dioxide itself

A gas inlet and gas outlet of the elastic sealing enclosure member may have the same structure as with the aforementioned rigid sealing enclosure member, but alternatively it is possible to seal the skin or mucosa with a tube, a bag or the like, and discharge the air therein as much as possible in advance, and then insert the tube 3a in from the orifice of the elastic sealing enclosure member having the form of a tube, a bag or the like such that air is prevented from entering therein as much as possible, and inject carbon dioxide (see Figs. 6 and 7).

In the case that the sealing enclosure member 2 is one made of a foldable sheet- or film-like material (hereinafter referred to as a 'sheet-type sealing enclosure member'), this can be used in the form of a tube, a bag or the like, with the skin or mucosa being sealed by binding the orifice of the tube, the bag or the like covering the desired part, or providing a retractable opening/closing orifice in which rubber, a spring or the like is placed in the orifice.

Moreover, in the case of a tubular sheet-type sealing enclosure member for which the part of application is made to be, for example, an arm (or a leg), it is possible to make the circumference of the tube be greater than that of the arm, seal the arm and fold the sealing enclosure member, so as to discharge air therein as much as possible in advance, and then use the sealing enclosure member with adhering to the skin or mucosa. A gas inlet and gas outlet of the sealing enclosure member may be as with the rigid sealing enclosure member, but alternatively it is possible to seal the skin or mucosa with the tube, bag or the like, and discharge the air therein as much as possible in advance, and then insert a tube in from the orifice of the sheet-type sealing enclosure member having the form of a tube, a bag or the like such that air is prevented from entering therein as much as possible, and inject carbon dioxide.

With the carbon dioxide external administration device 1, there are no particular limitations on the carbon dioxide supply means 3; for example, a commercially available carbon dioxide gas cylinder or the like can be used. Moreover, there may be used, for example, a device that uses a closed vessel having a tube attached thereto, wherein so-called dry ice, which is solid carbon dioxide, is vaporized inside the vessel, or carbon dioxide is generated inside the vessel through reaction between a carbonate and an acid, and the like.

With the carbon dioxide external administration device 1, regarding the tube 3a connecting the sealing enclosure member 2 to the carbon dioxide supply means 3, there are no particular limitations so long as the tube is such that gas will not leak out, and any material from which a tube can be formed, for example, rubber, a resin, metal or glass, may be used.

With the carbon dioxide external administration device 1, there are no particular limitations on a sheet-like product used for the carbon dioxide absorption aid 4, so long as this sheet-like product can be impregnated with a medium which dissolves carbon dioxide and can be patched on skin or mucosa; examples thereof include woven and nonwoven cloths made of natural fibers, synthetic fibers, semi-synthetic fibers or the like, semi-permeable membranes such as cellulose membranes, and hydrogel sheets made of a natural polymer, a synthetic polymer, a semi-synthetic polymer or the like, and one or more of these may be used.

Examples of the medium which dissolves carbon dioxide include water, alcohol, fat, wax and the like and one or more of these may be used.

Water having pH 7 to pH 2 is preferable, and acidic water having pH 6.5 to pH 4 is more preferable, since carbon dioxide is transdermally absorbed efficiently when it is dissolved in acidic water having pH 4 or more. In order to acidify water, a method of adding an organic acid or an inorganic acid into water is exemplified or acidic electrolyzed water may be used.

Regarding the alcohol, hard-to volatile alcohol at room temperature is preferable, and a polyhydric alcohol is more preferable. A volatile alcohol at room temperature is not preferable, since the skin temperature is lowered by the heat of vaporization and an increasing effect of carbon dioxide on blood flow is decreased.

Regarding the fat and wax, those that are liquid or semisolid at room temperature and impregnated with a sheet-like product may be used.

There are no particular limitations on the medium which dissolves carbon dioxide, but water-containing medium is preferable, and those containing water as much as possible and capable of supplying water to the skin are more preferable. An aqueous solution, suspension or swelling liquid of a thickener is preferably used as the liquid, since necessary viscosity is obtained with a little amount of the liquid, and a contact and adhesion to the skin is excellent. There are no particular limitations in a method for preparing these materials, which are indeed prepared by a known method.

As the thickener used in the viscous material, one or more selected from the group consisting of natural polymers, semi-synthetic polymers, synthetic polymers and inorganic materials can be used.

Examples of natural polymers include plant-derived polymers such as gum arabic, carrageenan, galactan, agar, xanthan gum, quince seed gum, guar gum, tragacanth, pectin, mannan, locust bean gum, wheat starch, rice starch, corn starch and potato starch; microorganism-derived polymers such as curdlan, xanthan gum, succinoglucan, dextran, hyaluronic acid and pullulan; and protein-type polymers such as albumin, casein, collagen, gelatin and fibroin; and one or more of these can be used. Among them, from the standpoint of affinity with skin and mucosae and so on, gum arabic, carrageenan, xanthan gum, tragacanth, hyaluronic acid, pullulan, pectin, mannan and locust bean gum can be preferably used, and from the standpoint of feeling in use and so on, carrageenan and pectin can be more preferably used.

Examples of semi-synthetic polymers include cellulosic polymers such as ethylcellulose, carboxymethylcellulose, carboxymethylethylcellulose, carboxymethyl starch, croscarmellose, crystalline cellulose, cellulose acetate, cellulose acetate phthalate, hydroxyethylcellulose, hydroxypropylcellulose, hydroxypropylmethylcellulose, hydroxypropylmethylcellulose phthalate, methylcellulose and methylhydroxypropylcellulose; starch-type polymers such as pregelatinized starch, partially pregelatinized starch, carboxymethyl starch, dextrin, methyl starch, starch-acrylic acid copolymers and cellulose-acrylonitrile graft copolymers; alginate-type polymers such as sodium alginate and propylene glycol alginate, and other polysaccharide-type polymers such as chondroitin sulfate sodium and sodium hyaluronate; and one or more of these can be used.

Among them, from the standpoint of affinity with skin and mucosae and so on, sodium alginate, propylene glycol alginate, sodium carboxymethylcellulose, dextrin and sodium hyaluronate can be preferably used, and from the standpoint of feeling in use and so on, sodium alginate, propylene glycol alginate and sodium hyaluronate can be more preferably used.

Examples of synthetic polymers include carboxyvinyl polymers, sodium polyacrylate, polyamines, polyacrylamide, polyvinylacetal diethylaminoacetate, polyvinyl alcohol, polyvinyl pyrrolidone, polyethylene glycol, polyethylene oxide, poly(meth)acrylic acid, polyvinyl methyl ether and so on; one or more of these can be used. Among them, from the standpoint of affinity with skin and mucosae and so on, carboxyvinyl polymers, sodium polyacrylate, polyacrylamide, polyvinyl alcohol and polyvinyl pyrrolidone can be preferably used, and from the standpoint of feeling in use and so on, polyvinyl alcohol, polyvinyl pyrrolidone and carboxyvinyl polymers can be more preferably used.

Examples of inorganic materials include hydrated silicon dioxide, light anhydrous silicic acid, colloidal alumina, bentonite, laponite and so on; and one or more of these can be used.

With the carbon dioxide external administration device 1, there are no particular limitations on an alcohol having a high vaporization temperature used in the carbon dioxide absorption aid 4, so long as this alcohol is liquid or semi-solid at room temperature and does not readily vaporize at the skin temperature of a human; examples thereof include monohydric alcohols such as isopropyl alcohol and 1-butanol, and polyhydric alcohols such as ethylene glycol, diethylene glycol, polyethylene glycol, propylene glycol, dipropylene glycol, 1,3-butanediol, 1,2-pentanediol, isoprene glycol, glycerol, diglycerol, triglycerol and tetraglycerol; and one or more of these can be used. There are no particular limitations on the method of using the alcohol; in accordance with the physical properties thereof and so on, the alcohol may be applied or sprayed onto the skin or mucosa as is, or impregnated or the like into a nonwoven cloth or the like and then patched on the skin.

With the carbon dioxide external administration device 1, there are no particular limitations on oil and fat used in the carbon dioxide absorption aid 4, so long as the oil and fat is liquid or semi-solid at room temperature and can be applied relatively thinly onto the skin or mucosa; examples thereof include natural vegetable oils and fats such as avocado oil, avocado butter, olive oil, sesame oil, safflower oil, soybean oil, camellia oil, sunflower oil and macadamia nut oil, and animal oils and fats such as squalane, mink oil, beef tallow, lard, chicken fat and horse fat; and one or more of these can be used.

There are no particular limitations on the method of using oil and fat; in accordance with the physical properties thereof and so on, the oil and fat may be applied or sprayed onto the skin or mucosa as is, or impregnated or the like into a nonwoven cloth or the like and then patched on the skin. With the carbon dioxide external administration device 1, there are no particular limitations on a wax used in the carbon dioxide absorption aid 4, so long as the wax is liquid or semi-solid at room temperature and can be applied relatively thinly onto the skin or mucosa; examples thereof include jojoba oil, carnauba wax, candelilla wax, beeswax and lanolin; and one or more of these can be used.

There are no particular limitations on the method of using the wax; in accordance with the physical properties thereof and so on, the wax may be applied or sprayed onto the skin or mucosa as is, or impregnated or the like into a nonwoven cloth or the like and then patched on the skin.

The carbon dioxide absorption aid 4 may be prepared from a single material and used, but an acidic viscous composition is preferable from the standpoint of an excellent contact and adhesion to the skin. As the acidic viscous composition, those comprising an acid, a thickener and water at least are preferable. As to the acidity, those having pH 4 to pH 6.5 are preferable, since carbon dioxide is dissolved in the molecular state therein and transdermal absorption rate is highly effective.

With the carbon dioxide external administration device 1, when the carbon dioxide absorption aid 4 is applied to the skin or mucosa, it is preferable to form a film of the carbon dioxide-dissolving medium on the skin or mucosa as thin as possible. When the film of the carbon dioxide-dissolving medium is too thick, it takes time for the carbon dioxide to dissolve in the medium, and to diffuse and be absorbed into the skin or mucosa, and hence the carbon dioxide absorption efficiency may be poor. However, in the case that the vaporization temperature of the carbon dioxide-dissolving medium is relatively low, when the film of the carbon dioxide-dissolving medium formed is too thin, the medium may vaporize or evaporate due to the skin temperature and hence may be lost from the skin or mucosa while the carbon dioxide is being absorbed; the amount of the carbon dioxide-dissolving medium to be used must thus be suitably adjusted.

Raw materials commonly used in external preparations and cosmetics, for example fragrances, colorants, surfactants, oils, moisturizers, alcohols, preservatives, antioxidants, sequestering agents, anti-colorants, ultraviolet absorbing/scattering agents, vitamins, amino acids, drugs such as arbutin, kojic acid, nutrients, anti-inflammatories, vasodilators, hormones, astringents, antihistamines, microbicides, sebum inhibiting agents, keratin stripping/dissolving agents, anti-seborrheic agents and antipruritics, and so on, may be blended into the carbon dioxide absorption aid 4, whereby the carbon dioxide absorption aid 4 can be used yet more suitably for a cosmetic or medical purpose.

With the carbon dioxide external administration device 1 used in the present invention, the carbon dioxide used is gaseous, and the proportion of carbon dioxide in the gas is preferably not less than 10 %, more preferably not less than 30 %. There is no upper limit in the ratio. Administration period of carbon dioxide is preferably longer than 5 minutes, more preferably longer than 10 minutes. There is no upper limit in the administration period.

The carbon dioxide external administration device (2):

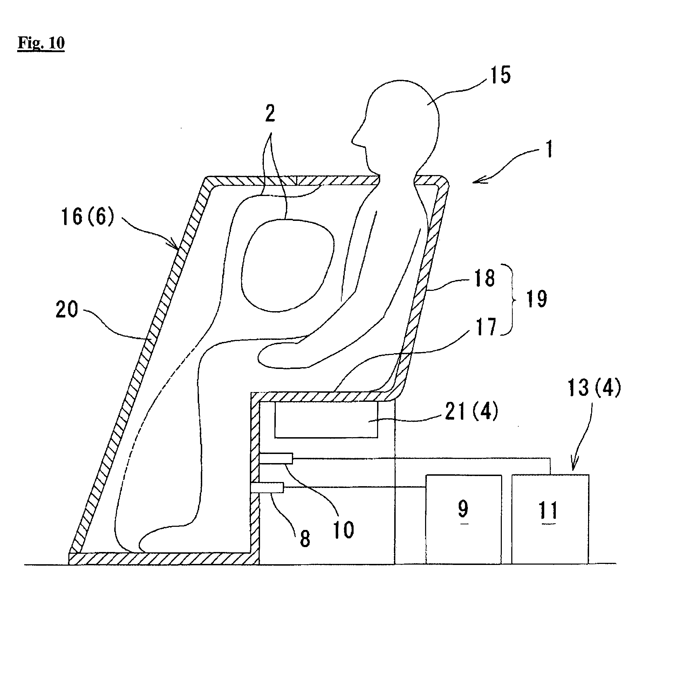

A carbon dioxide external administration device 1 used in the present invention is shown in Figure 10.

As shown in Fig. 10, in the administration device 1 of the present embodiment, the pressurizing enclosure member 6 is a hard and hollow box body 16, and is capable of accommodating the body of a treated person 15 entirely below his or her neck. The box body 16 includes: a main body part 19 having a seat part 17 and a backrest part 18; and a front lid part 20 for sealing off a front opening of the main body part 19 in an openable and closable manner.

The box body 16 is provided at its inner face side with the sealing enclosure member 2. The sealing enclosure member 2 is a bag body made of flexible synthetic resin, and is capable of accommodating the body of the treated person 15 entirely below his or her neck.

The first coupler 8 and the second coupler 10, each having a check valve function, are attached to the lower part of the box body 16. Further, from the first tank 9 connected to the first coupler 8, carbon dioxide can be supplied to the inside of the sealing enclosure member 2. Furthermore, from the second tank 11 connected to the second coupler 10, air can be supplied to a space formed between the sealing enclosure member 2 and the pressurizing enclosure member 6.

In the present embodiment, at a lower face of the seat part 17 of the box body 16, a heating member 21 including a heating wire heater or the like is provided. The heating member 21 can heat the inside of the sealing enclosure member 2 to a temperature exceeding the skin temperature. It should be noted that the skin temperature of the treated person is at a low temperature (about 25 °C) which is generally lower than the body temperature (about 36 °C), and therefore, it is only necessary for the heating member 21 to be capable of heating the inside of the sealing enclosure member 2 to a temperature exceeding the above mentioned low temperature.

When the administration device 1 of the present embodiment is used, as shown in Fig. 10, the treated person 15 is allowed to sit on the seat part 17 of the box body 16 with only his or her neck exposed to the outside, and the front lid part 20 is closed to airtightly close the box body 16.

In this state, carbon dioxide is supplied from the first tank 9 to the inside of the sealing enclosure member 2, and at the same time, air is supplied from the second tank 11 to the space formed between the sealing enclosure member 2 and the pressurizing enclosure member 6.

As a result, the inside of the sealing enclosure member 2 is almost filled with carbon dioxide, and the sealing enclosure member 2 is pressurized from outside by the air pressure of the inside of the pressurizing enclosure member 6, thus pressurizing the gas inside the sealing enclosure member 2 by a pressure exceeding atmospheric pressure. Therefore, also in the present embodiment, the pressurizing enclosure member 6 and the second tank 11 for supplying air to the inside of the pressurizing enclosure member 6 constitute the pressurizing member 13 for pressurizing the gas inside the sealing enclosure member 2 to a pressure exceeding atmospheric pressure.

As apparent from the examples described later, if the pressure of the carbon dioxide-containing gas, which is brought into contact with the body surface, is pressurized to a pressure exceeding atmospheric pressure as described above, a sensation of warmth of the entire body below the neck, serving as a region to be treated, is considerably intensified, thereby causing perspiration of the treated person 15. Accordingly, the pressurizing member 13 of the present embodiment also functions as the perspiration promoting means 4 for promoting perspiration at the body surface inside the sealing enclosure member 2.

Furthermore, the inside of the sealing enclosure member 2 is heated to a temperature exceeding the skin temperature by the heating member 21 provided at the seat part 17, thus facilitating the perspiration of the treated person 15. Therefore, in the present embodiment, the heating member 21 also functions as the perspiration promoting means 4 for promoting perspiration at the body surface.

Carbon dioxide dissolves in the sweat of the body surface, caused by the perspiration of the treated person 15 himself or herself, and this dissolved carbon dioxide is effectively absorbed transdermally and transmucosally. That is, since the sweat consequently serves as an absorption aid for carbon dioxide, antitumor effect can be obtained.

As described above, the administration device 1 of the present embodiment is also capable of obtaining antitumor effect regardless of whether or not the absorption aid is provided in advance inside the sealing enclosure member 2.

Further, in the present embodiment, the pressurizing enclosure member 6 surrounds the periphery of the sealing enclosure member 2 and air is supplied to the space between both of the enclosure members 2 and 6; therefore, the gas inside the sealing enclosure member 2 can be pressurized even if the supply amount of carbon dioxide is not increased. Therefore, as compared with the case where pressurization is carried out by increasing the supply amount of carbon dioxide, the inside of the sealing enclosure member 2 can be more inexpensively pressurized, and treatment by transdermal and transmucosal absorption of carbon dioxide can be more inexpensively carried out.

Next, the carbon dioxide external preparation, which is one of the means for absorption of carbon dioxide into the body in the present invention, is explained below.

Examples of the medium to dissolve carbon dioxide contained in the carbon dioxide external preparation include water, alcohol, fat and wax, and one or more of these materials may be used. Examples of water, alcohol, oil and fat, and wax listed in the carbon dioxide external administration device can be used in the same manner.

A method for preparing the carbon dioxide external preparation is exemplified as follows:

1) A method for dissolving carbon dioxide in the medium by directly blow carbon dioxide therein;
2) A composition for preparing external carbon dioxide agent, in which carbon dioxide is generated in a medium to dissolve carbon dioxide by reacting an acid and a carbonate salt in the medium; and
3) A composition for preparing external carbon dioxide agent, comprising microorganism to generate carbon dioxide through fermentation in a medium to dissolve carbon dioxide, and materials necessary for the fermentation,

are exemplified.

Carbon dioxide dissolved in a medium is easily dissipated in the air and difficult to preserve it. Also, there is a limitation in the amount of carbon dioxide dissolved in the medium. Therefore, it is preferable to use the composition for preparing external carbon dioxide agent, in which carbon dioxide is continuously generated by the reaction between an acid and a carbonate salt in the medium to dissolve carbon dioxide, and the external agent is prepared at use.

Examples of the composition for preparing external carbon dioxide agent, in which carbon dioxide is generated by the reaction between an acid and a carbonate salt in the medium to dissolve carbon dioxide, are shown below;

a) a composition for preparing external carbon dioxide agent consisting of a solid containing an acid and liquid containing a carbonate salt, in which the granular material and the liquid are mixed at use,
b) a composition for preparing external carbon dioxide agent consisting of a solid containing a carbonate salt and liquid containing an acid, in which the granular material and the liquid are mixed at use,
c) a composition for preparing external carbon dioxide agent, in which water is added to a solid containing an acid and a carbonate salt and mixed at use, and
d) a composition for preparing external carbon dioxide agent consisting of liquid containing an acid and liquid containing a carbonate salt, in which these liquids are mixed at use.

More specifically, the following compositions for preparing external carbon dioxide agent are exemplified;

e) a composition for preparing a carbon dioxide external agent characterized by comprising a substance generating an acid after being hydrolyzed, a carbonate, a thickener, and water as essential components, and further a gelating agent gelated by calcium ion, and a water-insoluble or poorly soluble calcium salt (WO 2006/80398 A);
f) a composition for preparing an external carbon dioxide agent comprising a granular material containing a water-soluble acid, a thickener and a water-soluble dispersant as the essential components wherein the said thickener is mixed with the water-soluble acid and the water-soluble dispersant; and a carbonate, water and a thickener as the essential components, which are to be mixed with the said granular material at use (WO 2002/80941),
g) a composition for preparing carbon dioxide gel for external use characterized by comprising granular material (A) and viscous material (B) to be mixed with the granular material (A):

(A) a granular material comprising a weak acid and a calcium ion trapping agent as essential components;
(B) a viscous material comprising calcium carbonate, a gelling agent to be gelated with calcium ions and water as essential components, and

h) a material for formation of carbon dioxide external preparation characterized by comprising a base agent that comprises a polymeric three-dimensional network structure impregnated with a viscous material containing at least an acid and water, and is made to contact with skin at use, and a reactant that contains at least a carbonate, and is made to contact with the base agent at use so as to generate carbon dioxide, the carbon dioxide being dissolved in the viscous material substantially in a non-bubble state.

The carbon dioxide external agent prepared from e) a composition for preparing a carbon dioxide external agent above (hereinafter referred to a composition for preparing a carbon dioxide external agent [1]) is explained in detail:

The composition for preparing a carbon dioxide external agent [1] used in the present invention comprises a substance generating an acid after being hydrolyzed, a carbonate, a thickener, and water as essential components.
The substance generating an acid after being hydrolyzed in the present invention is preferably one or more selected from lactone, a cyclic dimer of organic acid, and acid anhydride, and more preferably one or more selected from the crystalline forms of lactone, a cyclic dimer of organic acid, and acid anhydride. Specific examples include glucono-delta-lactone; pantolactone; D,L- or L-lactide (3,6-dimethyl-1,4-dioxane-2,5-dione); D,L- or L-glycolide; phthalic anhydride; maleic anhydride; and succinic anhydride. One of these examples or a combination of two or more of these examples may be used. Maleic anhydride and succinic anhydride are preferably used in combination with other substances generating an acid after being hydrolyzed such as glucono-delta-lactone or D,L-lactide to control the generating rate of an acid and consequently to control the generating rate of carbon dioxide at user's option since maleic anhydride and succinic anhydride are easily hydrolyzed and quickly generate an acid.

Any carbonate which generates carbon dioxide by the reaction with an acid may be used as a carbonate, and examples of the carbonate include ammonium carbonate, ammonium bicarbonate, potassium carbonate, potassium bicarbonate, potassium sesquicarbonate, sodium carbonate, sodium bicarbonate, sodium sesquicarbonate, lithium carbonate, lithium bicarbonate, lithium sesquicarbonate, cesium carbonate, cesium bicarbonate, cesium sesquicarbonate, magnesium carbonate, magnesium bicarbonate, calcium bicarbonate, calcium carbonate, magnesium hydroxide carbonate, or barium carbonate. One or more of these carbonates can be used and a water-soluble carbonate such as sodium bicarbonate or sodium carbonate is preferable.

There are no particular limitations on thickeners used and one or more selected from natural polymers, semi-synthetic polymers, synthetic polymers, and inorganic materials can be used.

Among them, examples of neutral or alkaline thickeners include the followings.

Examples of the natural polymers above include plant-originated polymers such as gum arabic, carrageenan, galactan, agar, quince seed, guar gum, tragacanth gum, mannan, locust bean gum, wheat starch, rice starch, tara gum, corn starch, and potato starch; microorganism-originated polymers such as curdlan, xanthan gum, succinoglucan, dextran, and pullulan; and protein polymers such as albumin, casein, collagen, gelatin and fibroin; one or more of these polymers can be used.

Examples of the semi-synthetic polymers above include cellulose polymers such as ethylcellulose, carboxymethylcellulose and its salts, carboxymethylethylcellulose and its salts, carboxymethyl starch and its salts, croscarmellose and its salts, crystalline cellulose, cellulose acetate, cellulose acetate phthalate, hydroxyethylcellulose, hydroxypropylcellulose, hydroxypropylmethylcellulose, hydroxypropylmethylcellulose phthalate, methylcellulose, and methylhydroxypropylcellulose; starch polymers such as pregelatinaized starch, partially pregelatinaized starch, carboxymethyl starch, dextrin, and methyl starch; alginate polymers such as sodium alginate, potassium alginate, ammonium alginate and propylene glycol alginate; and other polysaccharide polymers such as sodium chondroitin sulfate and sodium hyaluronate; and one or more of these polymers can be used.

Examples of the synthetic polymers above include sodium polyacrylate, polyvinylacetaldiethylaminoacetate, polyvinyl alcohol, polyvinyl pyrrolidone, methacrylic acid-ethyl acrylate copolymer, methacrylic acid - ethyl methacrylate copolymer, ethyl methacrylate - trimethylammoniumethyl chloride methacrylate copolymer, dimethylaminoethyl methacrylate-methyl methacrylate copolymer and the like. One or more of these polymers can be used.

Examples of the inorganic materials above include hydrated silicon dioxide, colloidal alumina, bentonite, laponite and the like; and one or more of these polymers can be used.

Examples of acidic thickeners include the followings.

Alginic acid, pectin and hyaluronic acid are included as a natural polymer; carboxyvinyl polymer is included as a semi-synthetic polymer and light anhydrous silicic acid is included as an inorganic material; one or more of these polymers or a material can be used.

There are no particular limitations on water used and natural water, tap water, distilled water, purified water and the like can be used.

A source of carbon dioxide in the composition for preparing a carbon dioxide external agent is the substance generating an acid after being hydrolyzed and the carbonate. The substance generating an acid after being hydrolyzed is gradually hydrolyzed to generate an acid when contacted with water, and the acid reacts with the carbonate to generate carbon dioxide gradually. Next, the viscous material is formed by the thickener and the water included in the composition for preparing a carbon dioxide external agent as essential components. Since the rate of hydrolysis of the substance generating an acid after being hydrolyzed and of generating carbon dioxide subsequent to the hydrolysis is slower in the viscous material than in water, carbon dioxide is generated gradually and constantly, and it provides the carbon dioxide external agent in which the generated carbon dioxide is difficult to leak into the air.

In the composition for preparing a carbon dioxide external agent [1], the composition for preparing a carbon dioxide external agent is preferable, wherein a granular material is formed by components other than water (a substance generating an acid after being hydrolyzed, a carbonate and a thickener) (hereinafter, the composition for preparing a carbon dioxide external agent [1-1]), or one or more component(s) selected from a substance generating an acid after being hydrolyzed, a carbonate, a gelating agent gelated by calcium ion and a water-insoluble or poorly soluble calcium salt form(s) a granulated material, and further at least a thickener and water form a viscous material (hereinafter, the composition for preparing a carbon dioxide external agent [1-2]). In addition, a granular material in the present invention means solid material such as powder, fine granule, granule, crystal and the like or a mixture of these solid materials.

The composition for preparing a carbon dioxide external agent [1-1] above is comprised of a granular material, which was prepared in advance using an usual technique, comprising a substance generating an acid after being hydrolyzed, a carbonate and a thickener (hereinafter, the granular material [A]) and water. The granular material [A] is mixed with water to provide the carbon dioxide external agent at use.

The granular material [A] and water are kept without contact before use and the carbon dioxide external agent is easily obtained when the granular material [A] and water are mixed at use. The granular material [A] can be easily prepared when a substance generating an acid, a carbonate and a thickener are simply mixed for example. It is preferable that the substance generating an acid, the carbonate and the thickener are mixed as uniformly as possible.

A dispersant is preferably included in the granular material [A] so that the thickener is dissolved or uniformly dispersed in water when it is mixed with water by forming less so-called "DAMA" or "MAMAKO". The term "DAMA" or "MAMAKO" means an aggregate including no water inside, which is formed by aggregation of the solid thickener, and whose surface is covered with a dissolved or swelled viscous substance when the thickener is mixed with water.

There are no particular limitations on the dispersant above, so long as it is easily soluble in water, chemically stable and can be used in a form of granule, and examples thereof include a starch derivative such as pregelatinized starch, α-cyclodextrin and the like; a sugar derivative such as sugar, glucose, fructose, sucrose, lactose, xylitol, D-sorbitol, D-mannitol and the like; a polysaccharide such as pullulan, xanthan gum and the like; a cellulose derivative and salt thereof such as hydroxypropylcellulose, hydroxypropylmethylcellulose, carmellose calcium, carmellose sodium and the like; a synthetic polymer such as polyvinyl pyrrolidone; and urea etc; and one or more of these material(s) can be used.

Moreover, additives other than the above mentioned dispersant, which are commonly used in medicines or cosmetics, may be added so that the thickener is easily dissolved or uniformly dispersed in water.

In the composition for preparing a carbon dioxide external agent [1-2], it is preferable that the substance generating an acid after being hydrolyzed and/or the carbonate form(s) a granular material, and further at least the thickener and water form a viscous material, wherein the thickener is dissolved or uniformly dispersed in water.

If the thickener and water is not sufficiently mixed, the viscosity of the carbon dioxide external agent obtained is so deficient that the generated carbon dioxide leaks into the air and the amount of transdermally or transmucosally absorbed carbon dioxide may be decreased or the carbon dioxide external agent may droop down from the skin or mucosa. Therefore it is preferable that the thickener and water form a viscous material which is to be mixed with a granular material at use to prepare the carbon dioxide external agent having sufficient viscosity.

Examples of a combination between the granular material and the viscous material are as follows;

(1) a viscous material comprising a carbonate, a thickener and water (hereinafter, the viscous material [B]) and a granular material comprising a substance generating an acid after being hydrolyzed (hereinafter, the granular material [B]); and
(2) a viscous material comprising a thickener and water (hereinafter, the viscous material [C]) and a granular material comprising a substance generating an acid after being hydrolyzed and a carbonate (hereinafter, the granular material [C]).

In a case of the viscous material [B], wherein a thickener is blended in the viscous material combined with a carbonate, the neutral or alkaline thickener which is used in the composition for preparing a carbon dioxide external agent [1] described before is preferably used. The reason comes from the concern that a carbonate may react with the thickener to generate a carbon dioxide when preparing the viscous material if the thickener is acidic.

In a case of the viscous material [C], the same thickener used in the composition for preparing a carbon dioxide external preparation [1] (the neutral or alkaline, or acidic thickener above) can be used.

In the composition for preparing a carbon dioxide external agent [1], all the components are preferably mixed when the carbon dioxide external agent is at use in either case of the composition for preparing a carbon dioxide external agent [1-1] or the composition for preparing a carbon dioxide external agent [1-2].

When preparing the composition for preparing a carbon dioxide external agent [1-1] using a water-absorbing support material, the granular material [A] is preferably contained in the support material not being contacted with water before use. In such case, the carbon dioxide external agent can be used as a material for wound remedy or pack cosmetic, if only the water-absorbing support material is soaked in or moistened with water.

There are no particular limitations on the water-absorbing support material above, so long as it is possible to contain the granular material [A], having water absorbing capacity, and applicable to the skin. The water-absorbing support material may be any of a woven fabric, a nonwoven fabric, a sponge or the like, and can be selected as appropriate in accordance with the specific purpose, part of application and so on. Among them, a nonwoven fabric is light, and has excellent capacity of keeping the granular material and is thus particularly preferable.

The prepared carbon dioxide external agent is applied to the target part with thickness of 1 mm or more. The applied part may be covered with a plastic film etc. fixed by adhesive tape etc. so that the applied external agent is not moved from the original part. The carbon dioxide external agent is preferably applied for more than 5 minutes, and more preferably more than 10 minutes. There is no limitation in the applying period, but it is preferable to exchange it with a new carbon dioxide external agent after about 24 hours. The external agent is preferably applied once a week or more and there is no limitation in applying times. Concentration of carbon dioxide is preferably over 300 ppm and more preferably over 1,000 ppm.

In addition, water of carbonated spring including an artificial carbonated spring, and carbon dioxide-containing water vapor also available. The carbon dioxide-containing water vapor means vapor such as natural carbon dioxide-containing water vapor which bursts from the ground, which is used for treating hemorrhoid etc. in place like Eastern Europe; and white smoke of carbon dioxide-containing water vapor which is generated by putting dry-ice in the air. A method of injecting gaseous carbon dioxide can also be available.

Of course, it is not intended that the present invention is limited to these means for supplying carbon dioxide and any means which enable carbon dioxide to be absorbed into the targeted part in an amount required for reducing or eliminating tumors, or suppressing tumor metastasis. The above mentioned carbon dioxide external administration device is more preferable for a means of enabling carbon oxide to be absorbed into the living body.

In the case where the antitumor agent comprising carbon dioxide as an active ingredient of the present invention is used in combination with a surgical therapy, a chemotherapy, a radiation therapy or an immunotherapy, the absorption of carbon dioxide may be done at every time when each therapy (including a multidisciplinary therapy) is done, or it may be done on the day when each therapy (including a multidisciplinary therapy) is not done.

The surgical therapies used in combination with the antitumor agent comprising carbon dioxide as an active ingredient of the present invention include, for example, extended radical operation, reduction surgery, functional preservation operation, endoscopic surgery and celoscope surgery.

The radiation therapies used in combination with the antitumor agent comprising carbon dioxide as an active ingredient of the present invention include, for example, gamma-knife therapy wherein a subject is irradiated with gamma-ray and cyber-knife therapy wherein a subject is irradiated with X-ray, both of them are stereotactic radiosurgery using Linac (a linear accelerator).

The chemotherapies used in combination with the antitumor agent comprising carbon dioxide as an active ingredient of the present invention include administration of antitumor agent such as molecular-targeted drugs, alkylating agents, antimetabolites, plant alkaloids, antitumor antibiotics, platinum agents, hormonal agents and biological response modifiers.

The molecular-targeted drugs used in said combined chemotherapy include ibritumomab tiuxetan, imatinib, erlotinib, gefitinib, gemtuzumab ozogamicin, sunitinib, cetuximab, sorafenib, dasatinib, tamibarotene, trastuzumab, tretinoin, panitumumab, bevacizumab, bortezomib and rituximab.

The alkylating agents used in said combined chemotherapy include ifosfamide, cyclophosphamide, dacarbazine, temozolomide, nimustine, busulfan and melphalan.

The antimetabolites used in said combined chemotherapy include enocitabine, capecitabine, carmofur, gemcitabine, cytarabine, tegafur, tegafur-uracil, nelarabine, fluorouracil, fludarabine, pemetrexed, pentostatin and methotrexate.

The plant alkaloids used in said combined chemotherapy include irinotecan, etoposide, sobuzoxane, docetaxel, nogitekan, paclitaxel, vinorelbine, vincristine, vindesine and vinblastine.

The antitumor antibiotics used in said combined chemotherapy include actinomycin D, aclarubicin, idarubicin, epirubicin, daunorubicin, doxorubicin (adriamycin), pirarubicin, bleomycin, peplomycin, mitomycin C and mitoxantrone.

The platinum agents used in said combined chemotherapy include oxaliplatin, carboplatin, cisplatin and nedaplatin.

The hormonal agents used in said combined chemotherapy include anastrozole, exemestane, ethinyl estradiol, chlormadinone, goserelin, tamoxifen, bicalutamide, flutamide, buredonizoron, leuprorelin and letrozole.

The biological response modifiers used in said combined chemotherapy include interferon-$\alpha$, interferon-$\beta$, interferon-$\gamma$, interleukin 2, ubenimex, dried BCG and lentinan.

The immunotherapies used in combination with the antitumor agent comprising carbon dioxide as an active ingredient of the present invention include, for example, cytokine therapy and immunomodulator as an active immunotherapy as well as active lymphocyte therapy and advanced activated NK cell therapy and activated lymphocyte therapy as a passive immunotherapy (immune cell therapy).

An inhibitor of nucleic acid synthesis can be used for the chemotherapy used in combination with the antitumor agent comprising carbon dioxide as an active ingredient of the present invention. Adriamycin is preferable for the inhibitor of nucleic acid synthesis used for this therapy.

## EXAMPLES

The present invention is illustrated in more details by the following Examples. However, the present invention is not limited to these Examples.

Example 1: Antitumor effects (1) against the implanted tumors in nude mice (using the carbon dioxide external administration device as a means allowing carbon dioxide to be absorbed)

[Test Method]

An MFH cell line NaraH, which is the cell line of a malignant soft tissue tumor, was subcutaneously implanted in the back of the 10 nude mice of 5 weeks old at 12 million cells / mouse. After the implantation, body weight and tumor volume of each nude mouse were measured. The mice were divided into two groups so that the variation in tumor volumes and body weights are reduced. That is, one is carbon dioxide absorption group and the other is control group treated in the same way as carbon dioxide absorption group but without the carbon dioxide absorption treatment. A treatment was started on the 2nd day after the implantation. After anesthetizing all groups by ether inhalation, the treatment was done according to the patent document 7, that is, an absorption aid for carbon dioxide comprising 93.77 % by mass of water, 5 % by mass of glycerin, 0.65 % by mass of carbomer, 0.15 % by mass of sodium alginate, 0.15 % by mass of sodium dihydrogen phosphate, 0.18 % by mass of sodium hydroxide and 0.10 % by mass of methylparaben was applied 2 mm thick to the dorsal skin wherein a tumor has been implanted. In the carbon dioxide absorption group, the lower body of a mouse was covered to seal with polyethylene bag having 4 cm width and 4 cm length. Then, the bag was filled with about 100 % concentration of carbon dioxide by using liquid carbon dioxide in order to allow carbon dioxide to be absorbed into the skin for 10 minutes. The carbon dioxide absorption was done twice-weekly and the treatment was done for 14 days. The carbon dioxide absorption was done a total of 4 times. Tumor volume was calculated by the following equation:

$$\text{(Maximum diameter of implanted tumor mass)} \times \text{(Minimum diameter of implanted tumor mass)} \times \text{(Minimum diameter of implanted tumor mass)} \times 1/2.$$

The measurement of tumor volume was done on the 2nd, 7th, 9th, 14th and 16th day after tumor implantation.

Test Results

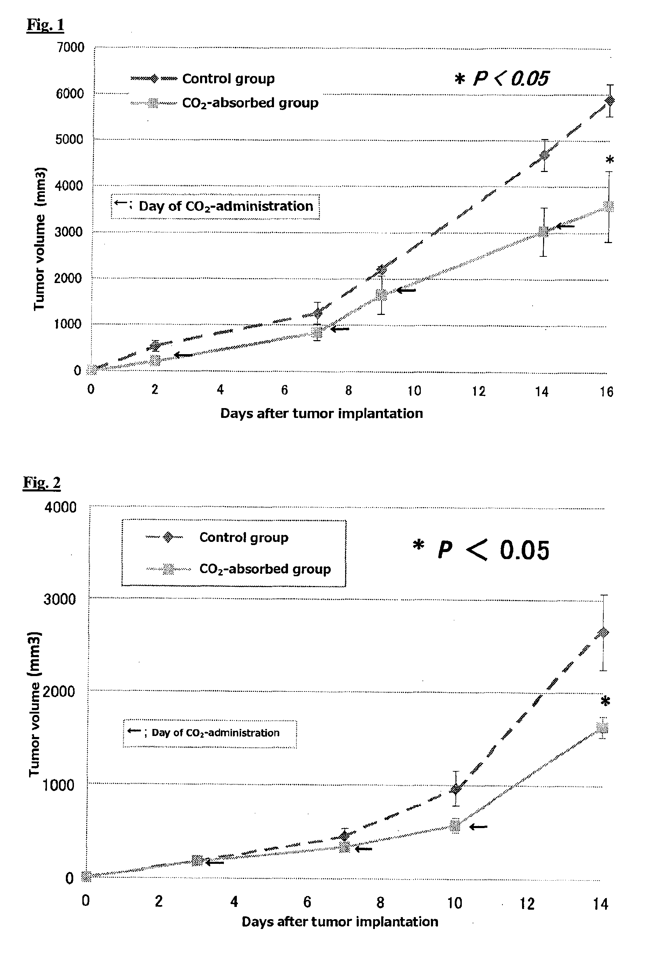

It was shown by t-test that tumor volume of the carbon dioxide absorption group was statistically significantly reduced compared to the control group on the 16th day after the tumor implantation (Fig. 1). During the experiment period, there were no deaths in the two groups.

Example 2: Antitumor effects (2) against the implanted tumors in nude mice (using the carbon dioxide external administration device as a means allowing carbon dioxide to be absorbed)

Test Method

An MFH cell line NaraH, which is the cell line of a malignant soft tissue tumor, was subcutaneously implanted in the back of the 12 nude mice of 5 weeks old at 12 million cells / mouse. After the implantation, body weight and tumor volume of each nude mouse were measured. The mice were divided into 2 groups so that the variation in tumor volumes and body weights are reduced. That is, one is carbon dioxide absorption group and the other is control group treated in the same way as carbon dioxide absorption group but without the carbon dioxide absorption treatment. The treatment according to Example 1 was started on the 3rd day after the implantation. The carbon dioxide absorption was done twice-weekly and the treatment was done for 14 days. The carbon dioxide absorption was done a total of 3 times. Tumor volume was calculated by the following equation:

$$\text{(Maximum diameter of implanted tumor mass)} \times \text{(Minimum diameter of implanted tumor mass)} \times \text{(Minimum diameter of implanted tumor mass)} \times 1/2.$$

The measurement of tumor volume was done on the 3rd, 7th, 10th, and 14th day after tumor implantation.

Test Results

It was shown by t-test that tumor volume of the carbon dioxide absorption group was statistically significantly reduced compared to the control group on the 14th day after tumor implantation (Fig. 2). During experimental period, there were no deaths in the two groups.

Example 3: Antitumor effects (3) against the implanted tumors in nude mice (using the carbon dioxide external administration device as a means allowing carbon dioxide to be absorbed)

Test Method

An MFH cell line NaraH, which is the cell line of a malignant soft tissue tumor, was subcutaneously implanted in the back of the 20 nude mice of 5 weeks old at 12 million cells / mouse. After the implantation, body weight and tumor volume were measured. The mice were divided into the following 3 groups so that the variation in tumor volumes and body weights are reduced. The treatment according to Example 1 was started on the 3rd day after the implantation (Table 1).

Table 1

| Test groups | Number of mice | Experiment contents |
|---|---|---|
| Group of twice-weekly administration | 5 | Carbon dioxide absorption was done twice-weekly |
| Group of 5 times-weekly administration early in treatment | 5 | Carbon dioxide absorption was done in 5 consecutive days from the first day of the treatment (3 days after tumor implantation). |
| Control group | 5 | The mice were anesthetized by ether and an absorption aid for carbon dioxide of Example 1 was applied to the tumor-implanted dorsal skin. However, the carbon dioxide absorption was not done. |

The treatment was done for 16 days. Tumor volume was calculated by the following equation: (Maximum diameter of implanted tumor mass) x (Minimum diameter of implanted tumor mass) x (Minimum diameter of implanted tumor mass) x 1/2. The measurement of tumor volume was done on the 3rd, 7th, 10th, 14th and 16th day after tumor implantation.

Test Results

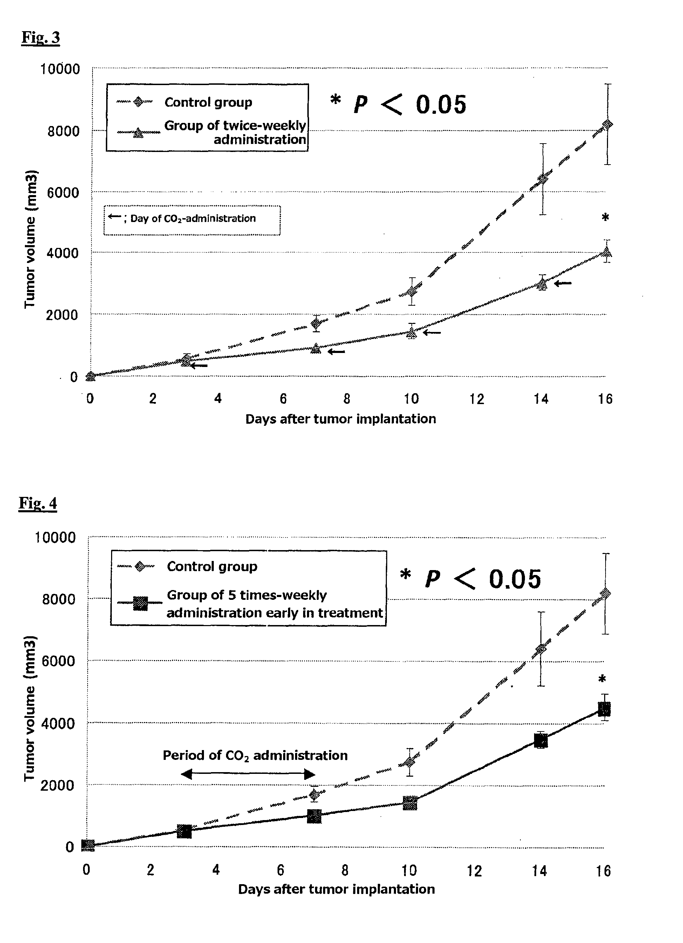

It was shown by t-test that tumor volumes of the group of twice-weekly administration and the group of 5 times-weekly administration early in treatment were statistically significantly reduced compared to the control group (Fig. 3 and Fig. 4).

Example 4: Combined effects with a chemotherapeutic agent against the implanted tumors in nude mice (using the carbon dioxide external administration device as a means allowing carbon dioxide to be absorbed)

Test Method

A cell line NaraH, which is the cell line of a malignant soft tissue tumor, was subcutaneously implanted in the back of the 17 nude mice of 5 weeks old at 12 million cells / mouse. After the implantation, body weight and tumor volume of each nude mouse were measured. The mice were divided into the following 3 groups so that the variation in tumor volumes and body weights are reduced. The treatment according to Example 1 was started on the 3rd day after the implantation (Table 2).

Table 2

| Test groups | Number of mice | Experiment Contents |
|---|---|---|
| Carbon dioxide-alone group | 4 | Only carbon dioxide absorption was done twice-weekly. |
| Chemotherapeutic agent-alone group | 5 | The mice were given 6 mg/kg of adriamycin and were anesthetized by ether, and an absorption aid for carbon dioxide of Example 1 was applied to the mice. However, the carbon dioxide absorption was not done. |
| Combination group | 5 | The mice were given 6 mg/kg of adriamycin and were anesthetized by ether. Then, the carbon dioxide absorption was done as with Example 1. |
| Control group | 5 | The mice were anesthetized by ether and an absorption aid for carbon dioxide of Example 1 was applied to the tumor-implanted dorsal skin. However, the carbon dioxide absorption was not done. |

The treatment was done for 9 days. Tumor volume was calculated by the following equation: (Maximum diameter of implanted tumor mass) x (Minimum diameter of implanted tumor mass) x (Minimum diameter of implanted tumor mass) x 1/2. The measurement of tumor volume was done on the 3rd, 7th and 9th day after tumor implantation.

[Test Results]

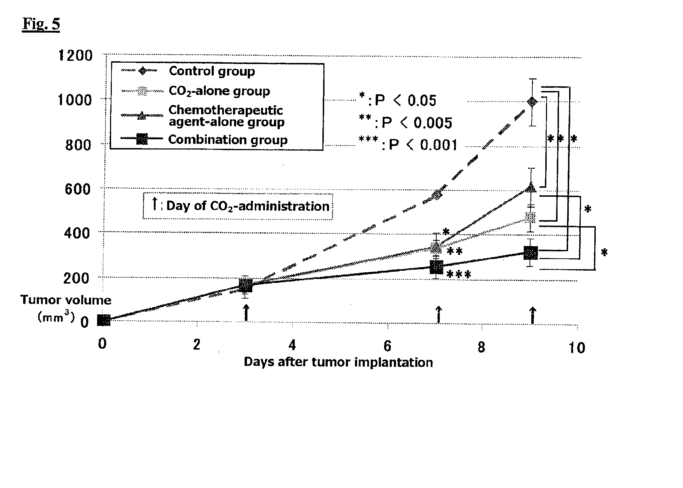

A summary of the results is shown in Figure 5. It was shown by t-test that tumor volumes of the carbon dioxide-alone group, the chemotherapeutic agent-alone group and the combination group were statistically significantly reduced compared to the control group on the 7th day after the tumor implantation (Table 3).

Table 3

| Test groups | Significance level for control group |
|---|---|
| Carbon dioxide-alone group | P < 0.001 |
| Chemotherapeutic agent-alone group | P < 0.005 |
| Combination group | P < 0.05 |

It was shown by t-test that tumor volumes of the carbon dioxide-alone group, the chemotherapeutic agent-alone group and the combination group were statistically significantly reduced compared to the control group on the 9th day after the tumor implantation. In addition to that, it was shown by t-test that tumor volume of the combination group was statistically significantly reduced compared to the carbon dioxide-alone group and the chemotherapeutic agent-alone group (Table 4).

Table 4

| Test groups | Significance level for control group | Significance level for carbon dioxide-alone group | Significance level for chemotherapeutic agent-alone group |
|---|---|---|---|
| Carbon dioxide-alone group | P < 0.05 | - | - |
| Chemotherapeutic agent-alone group | P < 0.05 | - | - |
| Combination group | P < 0.05 | P < 0.05 | P < 0.05 |

It is apparent from the above mentioned results that not only does the antitumor agent comprising carbon dioxide as an active ingredient of the present invention shows the therapeutic effect against tumor by monotherapy, but also it shows more excellent therapeutic effect against tumor in combination with a chemotherapeutic agent than when they are used alone.
Example 5: Combined effects with a chemotherapeutic agent against the implanted malignant breast carcinoma in nude mice (using the carbon dioxide external administration device as a means allowing carbon dioxide to be absorbed)

Test Method

MDA-MB-231, a cell line of a malignant breast carcinoma, was subcutaneously implanted in the back of the 18 nude mice of 5 weeks old at 12 million cells / mouse. On the 53rd day after the implantation, body weight and tumor volume of each nude mouse were measured. The mice were divided into the following 4 groups so that the variation in tumor volumes and body weights are reduced. The treatment was done according to Example 1 (Table 5).

Table 5

| Test groups | Number of mice | Experiment contents |
|---|---|---|
| Carbon dioxide-alone group | 5 | Only carbon dioxide absorption was done twice-weekly. |
| Chemotherapeutic agent-alone group | 4 | The mice were given 4 mg/kg of adriamycin and were anesthetized by ether, and an absorption aid for carbon dioxide of Example 1 was applied to the mice. |

(continued)

| Test groups | Number of mice | Experiment contents |
|---|---|---|
|  |  | However, the carbon dioxide absorption was not done. |
| Combination group | 4 | The mice were given 4 mg/kg of adriamycin and were anesthetized by ether. Then, the carbon dioxide absorption was done as with Example 1. |
| Control group | 5 | The mice were anesthetized by ether and an absorption aid for carbon dioxide of Example 1 was applied to the tumor-implanted dorsal skin.<br>However, the carbon dioxide absorption was not done. |

The starting date of the test was defined as the 0 day and each treatment was done on the 1 st, 5th, 8th, 12th and 15th day. Tumor volume was calculated by the following equation: (Maximum diameter of implanted tumor mass) x (Minimum diameter of implanted tumor mass) x (Minimum diameter of implanted tumor mass) x 1/2. The measurement of tumor volume was done on the 8th and 15th day after tumor implantation. The measurement of body weights of the nude mice as an indication of a side effect was done on the 8th and 15th day after tumor implantation.

Test Results

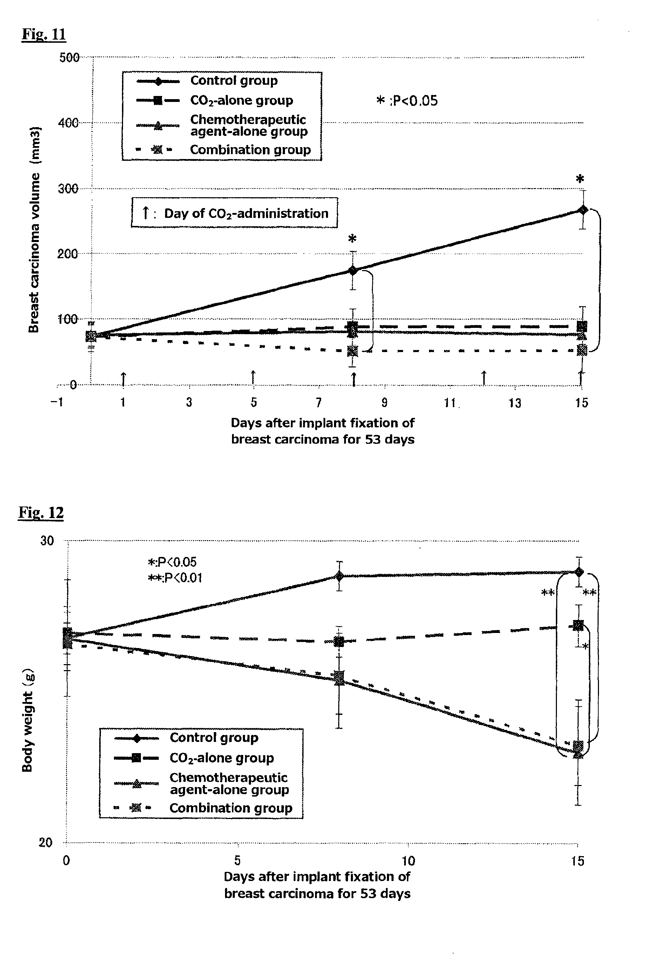

A summary of the results is shown in Figure 11. It was shown by t-test that tumor volumes of the combination group was statistically significantly reduced compared to the control group on the 8th day after tumor implantation ($P < 0.05$).

It was shown by t-test that tumor volumes of carbon dioxide-alone group, the chemotherapeutic agent-alone group and the combination group were statistically significantly reduced compared to the control group on the 15th day after tumor implantation (Table 6). It is striking that tumor was completely disappeared in two of the four mice in the combination group.

Table 6

| Comparative test goups | Significance level for control group |
|---|---|
| Carbon dioxide-alone group vs. Control group | $P < 0.001$ |
| Chemotherapeutic agent-alone group vs. Control group | $P < 0.005$ |
| Combination group vs. Control group | $P < 0.05$ |

Weight reduction as an indicator of a side effect was statistically significant in the chemotherapeutic agent-alone group compared to the control group ($P < 0.01$). However, significant weight reduction was not found in the carbon dioxide group. Significant weight reduction was found in the combination group compared to the control group ($P < 0.01$) and in the chemotherapeutic agent-alone group compared to the carbon dioxide-alone group ($P < 0.05$) (Figure 12). It is concluded by these results that no weight reduction of mice is caused by the administration of carbon dioxide.

It is apparent from the above mentioned results that not only does the antitumor agent comprising carbon dioxide as an active ingredient of the present invention show the therapeutic effect against tumor by monotherapy, but also it shows more excellent therapeutic effect against tumor in combination with a chemotherapeutic agent than when they are used alone, as well as there is little side effect.

Example 6: Combined effects with radiation against the implanted tumors in nude mice (using the carbon dioxide external administration device as a means allowing carbon dioxide to be absorbed)

Test Method

An MFH cell line NaraH, which is the cell line of a malignant soft tissue tumor, was subcutaneously implanted in the back of the 17 nude mice of 5 weeks old at 12 million cells / mouse. After the implantation, body weight and tumor volume of each nude mouse were measured. The mice were divided into the following 4 groups so that the variation in tumor volumes and body weights are reduced. The treatment according to Example 1 was started on the 3rd day after the implantation (Table 7).

Table 7

| Test groups | Number of mice | Experiment contents |
|---|---|---|
| Radiation-alone group | 6 | The mice were anesthetized by ether and Nembutal. The body other than tumor was protected by lead block and the tumor was exposed to 3 Gry of radiation, and then the mice were returned to their cages. |
| Combination group | 6 | After anesthetized by ether, the mice received carbon dioxide absorption as with Example 1. Then, the mice were anesthetized by Nembutal and were exposed to radiation as with the radiation-alone group. |
| Control group | 6 | The mice were anesthetized by ether and Nembutal, and an absorption aid for carbon dioxide of Example 1 was applied to them. However, the carbon dioxide absorption and radiation were not done. |

The radiation was done by an X-ray generator (MBR-1505R2, Hitachi Medical Corporation).

In addition, a test for an effect of treatment by carbon dioxide alone against the above mentioned tumor cells was omitted in this experiment because its effect has already been proved.

Test Results

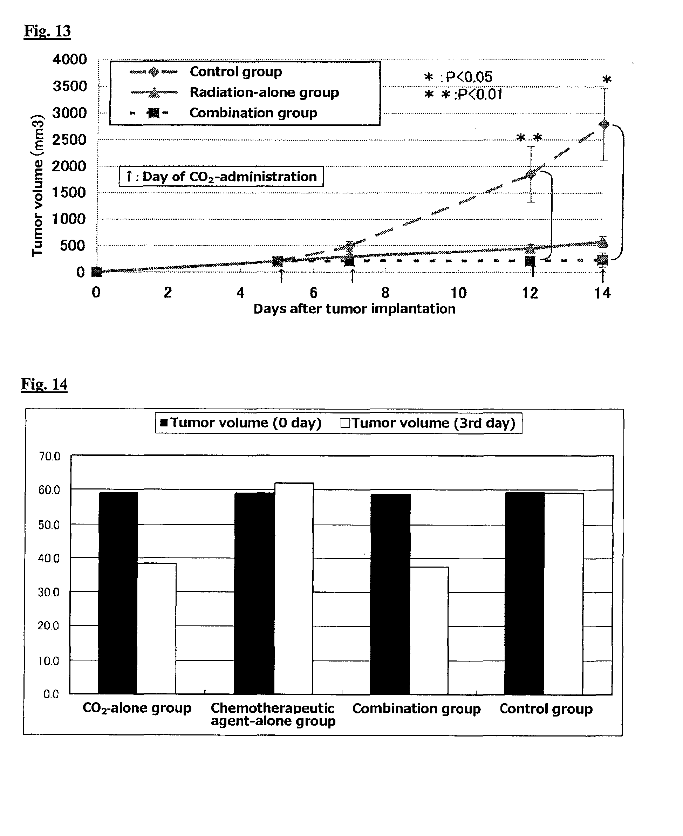

A summary of the results is shown in Figure 13. It was shown by t-test that tumor volumes of the combination group was statistically significantly reduced compared to the control group on the 12th day after the tumor implantation ($P < 0.01$). In addition, it was shown by t-test that tumor volumes of the combination group was statistically significantly reduced compared to the radiation-alone group ($P < 0.05$). It was shown by t-test that tumor volumes of the radiation-alone group was statistically significantly reduced compared to the control group ($P < 0.01$).

It is apparent from the above mentioned results that the antitumor agent comprising carbon dioxide as an active ingredient of the present invention shows an increased antitumor effect when used in combination with radiation.

Example 7: Antitumor effects (4) against the implanted tumors in nude mice (using the carbon dioxide external administration device as a means allowing carbon dioxide to be absorbed)

Test Method

A breast carcinoma cell line MDA-MB-231, which is the cell line of a malignant soft tissue tumor, was subcutaneously implanted in the back of the 23 nude mice of 5 weeks old at 20 million cells / mouse. On the 18th day after the implantation, body weight and tumor volume of each nude mouse were measured. The mice were divided into the following 4 groups so that the variation in tumor volumes and body weights are reduced. The treatment was done according to Example 1 (Table 8), but only once.

Table 8

| Test groups | Number of mice | Experiment contents |
|---|---|---|
| Carbon dioxide-alone group | 6 | The carbon dioxide absorption was done for 10 minutes. |
| Chemotherapy-alone group | 6 | The mice were given 3 mg/kg of adriamycin and were anesthetized by ether, and an absorption aid was applied to the mice. However, the carbon dioxide absorption was not done and the mice were returned to their cages. |
| Combination group | 5 | The mice were given 3 mg/kg of adriamycin and the carbon dioxide absorption was done for 10 minutes as with Example 1. |
| Control group | 6 | The mice were anesthetized by ether and an absorption aid for carbon dioxide of Example 1 was applied to the tumor-implanted dorsal skin. However, the carbon |
| | | dioxide absorption was not done. |

Tumor volume was calculated by the following equation: (Maximum diameter of implanted tumor mass) x (Minimum diameter of implanted tumor mass) x (Minimum diameter of implanted tumor mass) x 1/2. The measurement of tumor volume was done on the 3rd day after starting of the treatment.

Test Results

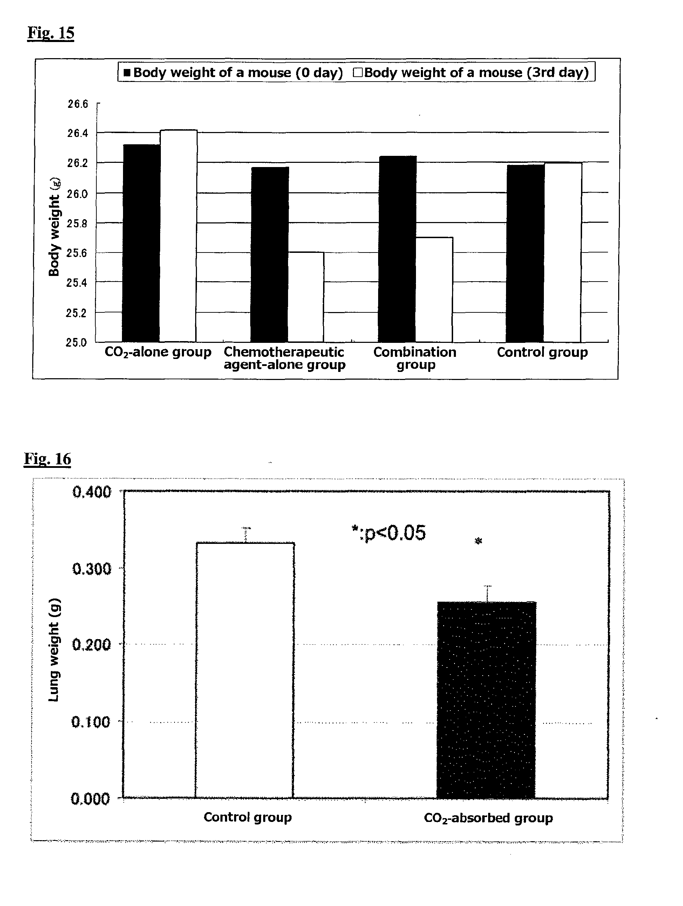

The tumor volumes in the carbon dioxide-alone group and the combination group were reduced to two-thirds of initial volumes thereof despite only for 10 minutes of the carbon dioxide absorption. The tumor volumes in the chemotherapy-alone group and the control group were not changed (Figure 14). The body weight reduction as an indicator of the side effect was not observed in the carbon dioxide-alone group and the control group. However, the body weights were reduced in the chemotherapy-alone group and the combination group (Figure 15).

It is apparent from the above mentioned results that the antitumor agent comprising carbon dioxide as an active ingredient of the present invention shows an antitumor effect even when administered only once and has an immediate effect.

Example 8: Effects against the implanted highly lung-metastatic tumors in nude mice (using the carbon dioxide external administration device as a means allowing carbon dioxide to be absorbed)

Test Method

A cell line LM-8, which is the cell line of a highly lung-metastatic tumor, was subcutaneously implanted in the back of the 11 nude mice of 5 weeks old at 1 million cells / mouse. On the 3rd day after the implantation, body weight and tumor volume of each nude mouse were measured. The mice were divided into two groups so that the variation in tumor volumes and body weights are reduced. That is, one is carbon dioxide absorption group (6 mice) and the other is control group (5 mice) treated in the same way as the carbon dioxide absorption group but without the carbon dioxide absorption treatment. The treatment according to Example 1 was started on the 3rd day after the implantation. The carbon dioxide absorption was done three times-weekly and the treatment was done for 14 days. The carbon dioxide absorption was done a total of 6 times. Tumor volume was calculated by the following equation: (Maximum diameter of implanted tumor mass) x (Minimum diameter of implanted tumor mass) x (Minimum diameter of implanted tumor mass) x 1/2. The measurement of tumor volume was done on the 3rd, 7th, 10th, 14th, 17th and 21 st day after the tumor implantation. On the 21 st day after the tumor implantation, a lung of each mouse was isolated and the lung weight was measured. The cross-sections of the lung, where the metastasized tumor have been stained by hematoxylin and eosin, were chosen at random and a proportion of the stained area in the cross-section was analyzed by using an image analyzing software ImageJ to obtain the lung metastasis rate of the tumor.

Test Results

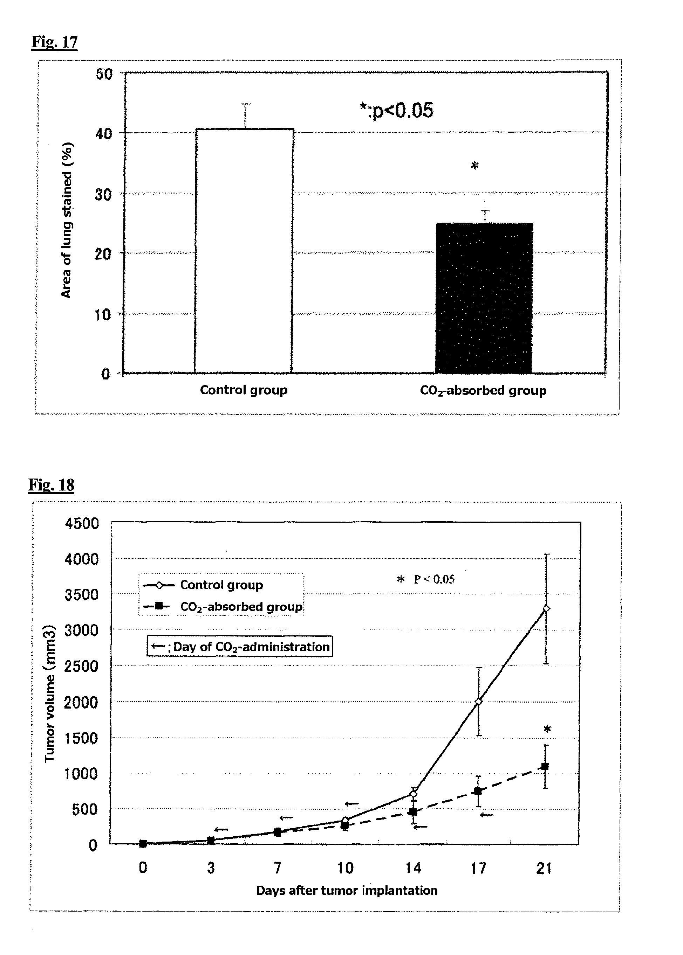

It was shown by t-test that lung weights ($P < 0.05$; Figure 16) as well as lung metastasis rates ($P < 0.05$; Figure 17) of the carbon dioxide absorption group were statistically significantly reduced compared to the control group on the 21 st day after the tumor implantation. In addition, it was shown by t-test that subcutaneously-implanted tumor volumes of the carbon dioxide absorption group was statistically significantly reduced compared to the control group ($P < 0.05$; Figure 18). During the experiment period, there were no deaths in the two groups.

It is apparent from the above mentioned results that not only does the antitumor agent comprising carbon dioxide as an active ingredient of the present invention show a tumor-reducing effect, but also it shows a metastasis-inhibiting effect.

INDUSTRIAL APPLICABILITY

The antitumor agent comprising carbon dioxide as an active ingredient of the present invention can reduce the tumor volume or eradicate the tumor and inhibit metastasis in a patient, who is difficult to take a surgical therapy, a chemotherapy, a radiation therapy or an immunotherapy, with little side effects. Further, the treatment by the antitumor agent of the present invention in combination with the above mentioned therapies can potentiate the treatment effect and reduce the side effect as compared with a monotherapy or a multidisciplinary therapy thereof.

**Claims**

1. An antitumor agent comprising carbon dioxide as an active ingredient.

2. The antitumor agent according to Claim 1,

wherein carbon dioxide is administered locally.

3. The antitumor agent according to Claim 2,
wherein carbon dioxide is administered transdermally through the skin near the tumor.

4. The antitumor agent according to Claim 2,
wherein carbon dioxide is administered transdermally through skin far from the tumor.

5. The antitumor agent according to Claim 1,
for inhibiting tumor metastasis.

6. The antitumor agent according to Claim 1,
wherein the agent is used in combination with one or more therapies selected from a surgical therapy, a chemotherapy, a radiation therapy and an immunotherapy.

7. The tumor is one or more tumors selected from malignant tumors of brain nerves such as glioma and meningioma; malignant tumors of oral cavity, nose, nasal cavity, larynx or pharynx such as tongue cancer, gum cancer, malignant lymphoma, malignant melanoma, upper jaw cancer, nose cancer, nasal cavity cancer, laryngeal cancer and throat cancer; malignant tumors of thyroid such as thyroid papillary carcinoma, follicular thyroid cancer and medullary thyroid carcinoma; malignant tumors of respiratory tract such as squamous cell carcinoma, adenocarcinoma, alveolar cell carcinoma, large cell anaplastic carcinoma, small cell anaplastic carcinoma and carcinoid; malignant tumors of breast such as breast cancer, mammary Paget's disease and breast sarcoma; malignant tumors of blood such as acute myeloid leukemia, acute promyelocytic leukemia, acute myelomonocytic leukemia, acute monocytic leukemia, acute lymphocytic leukemia, acute undifferentiated leukemia, chronic myeloid leukemia, chronic lymphocytic leukemia, adult T-cell leukemia, malignant lymphoma, multiple myeloma and primary macroglobulinemia; malignant tumors of digestive organ such as esophageal cancer, stomach cancer, stomach-large intestine leiomyosarcoma, stomach-intestine malignant lymphoma, pancreatic-gallbladder cancer, duodenal cancer, colon cancer, primary liver cancer and hepatoblastoma; malignant tumors of female genital such as uterine epithelium cancer, uterine cervix squamous epithelium cancer, uterine adenocarcinoma, uterine gland squamous epithelium cancer, uterine body adenoacanthoma, uterine sarcoma, uterine cancer sarcoma, invasive hydatidiform mole, malignant chorioepithelioma of uterus, malignant melanoma of uterus, ovarian cancer and mesodermal mixed tumor; malignant tumors of urinary organ such as kidney cancer, transitional cell cancer of renal pelvis, ureter transitional cell carcinoma, bladder papillary carcinoma, bladder transitional cell carcinoma, prostate cancer, urinary tract squamous cell carcinoma, urethral adenocarcinoma and Wilms' tumor; malignant tumors of musculoskeletal such as rhabdomyosarcoma, fibrosarcoma, osteosarcoma, chondrosarcoma, synovial sarcoma, mucus sarcoma, liposarcoma, Ewing sarcoma and multiple myeloma; malignant tumors of skin such as skin squamous cell carcinoma, basal cell skin cancer, skin Bowen's disease, Paget's disease of skin and cutaneous malignant melanoma; and malignant tumors of body cavity such as malignant mesothelioma cancer, malignant melanoma, metastatic adenocarcinoma, metastatic squamous cell carcinoma, metastatic sarcoma, leukemia, malignant lymphoma and neuroblastoma; or benign tumors of brain such as meningioma, pituitary adenoma and nerve sheath tumor; benign tumors of subcutaneous tissue such as nevus, suspended fibroma, hemangioma, vascular birthmark, lymphangioma, pyogenic granuloma, seborrheic keratosis, dermatofibroma, keratoacanthoma, keloid, lipoma, brown lipoma, neurofibromatosis, schwannoma and atheroma; benign tumors of bone such as osteocartilaginous exostosis, chondroma, chondroblastoma, osteoid osteoma and giant cell tumor; benign tumors of intestinal such as lipoma, fibroids and polyp; benign tumors of liver such as hepatocellular adenoma and bile duct adenoma; benign tumors of bile duct such as papilloma and villous adenoma; benign tumors of ear such as ear mushroom and cholesteatoma; and benign tumors of oral cavity or salivary gland such as benign pleomorphic adenoma, monomorphic adenoma, oncocytoma, papillary cystadenoma lymphoma and ameloblastoma.

**Fig. 1**

Tumor volume (mm3) vs Days after tumor implantation. Legend: Control group, CO$_2$-absorbed group. $* P < 0.05$. ←: Day of CO$_2$-administration.

**Fig. 2**

Tumor volume (mm3) vs Days after tumor implantation. Legend: Control group, CO$_2$-absorbed group. $* P < 0.05$. ← ; Day of CO$_2$-administration.

**Fig. 3**

**Fig. 4**

**Fig. 5**

Fig. 6

Fig. 7

**Fig. 8**

Fig. 9

Fig. 10

**Fig. 11**

**Fig. 12**

## Fig. 13

## Fig. 14

Fig. 15

Fig. 16

Fig. 17

Fig. 18

## INTERNATIONAL SEARCH REPORT

| | International application No. |
|---|---|
| | PCT/JP2012/057360 |

### A. CLASSIFICATION OF SUBJECT MATTER
*A61K33/00*(2006.01)i, *A61P35/00*(2006.01)i, *A61P35/02*(2006.01)i, *A61P43/00*(2006.01)i

According to International Patent Classification (IPC) or to both national classification and IPC

### B. FIELDS SEARCHED

Minimum documentation searched (classification system followed by classification symbols)
A61K33/00, A61P35/00, A61P35/02, A61P43/00

Documentation searched other than minimum documentation to the extent that such documents are included in the fields searched
Jitsuyo Shinan Koho          1922-1996   Jitsuyo Shinan Toroku Koho   1996-2012
Kokai Jitsuyo Shinan Koho    1971-2012   Toroku Jitsuyo Shinan Koho   1994-2012

Electronic data base consulted during the international search (name of data base and, where practicable, search terms used)
CA/MEDLINE/EMBASE/BIOSIS(STN), JSTPlus/JMEDPlus/JST7580(JDreamII)

### C. DOCUMENTS CONSIDERED TO BE RELEVANT

| Category* | Citation of document, with indication, where appropriate, of the relevant passages | Relevant to claim No. |
|---|---|---|
| X<br>A | TAKIGUCHI, S. et al., Surgical Endoscopy, 2000, Vol.14, p.41-44 | 1,2,5,6,7<br>3,4 |
| A | HOSKIN, P.J. et al., CANCER, 2005, Vol.103, No. 11, p.2287-2297 | 1-7 |
| A | JP 2006-63009 A  (Konica Minolta Medical & Graphic, Inc.),<br>09 March 2006 (09.03.2006),<br>entire text<br>(Family: none) | 1-7 |
| A | WO 2004/002393 A1  (Neo Chemir Inc.),<br>08 January 2004 (08.01.2004),<br>entire text; fig. 1 to 7<br>& US 2005/0254993 A1     & EP 1541111 A1<br>& CN 1665467 A | 1-7 |

☒  Further documents are listed in the continuation of Box C.          ☐  See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 10 May, 2012 (10.05.12) | 22 May, 2012 (22.05.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

| | International application No. |
|---|---|
| | PCT/JP2012/057360 |

C (Continuation).   DOCUMENTS CONSIDERED TO BE RELEVANT

| Category* | Citation of document, with indication, where appropriate, of the relevant passages | Relevant to claim No. |
|---|---|---|
| A | WO 2008/047829 A1  (Neo Chemir Inc.),<br>24 April 2008 (24.04.2008),<br>entire text; fig. 1 to 6<br>& US 2010/0305497 A1    & EP 2078520 A1 | 1-7 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000319187 A
- WO 200280941 A
- WO 200680398 A
- WO 200357228 A
- WO 200516290 A
- WO 200404745 A
- WO 200402393 A
- WO 2007112726 A
- JP 2009120606 A
- WO 2008047829 A